# EUROPEAN PATENT APPLICATION

(11) **EP 2 543 437 A2**
(43) Date of publication of application: **09.01.2013**
(21) Application number: 11750781.4
(22) Date of filing: 03.03.2011
(51) Int. Cl.: B01J 31/22, C07C 45/62, C07C 47/21, C07B 53/00, C07B 61/00

(54) **HOMOGENEOUS ASYMMETRIC HYDROGENATION CATALYST**

(30) Priority: 04.03.2010 JP 2010047741
(71) Applicant: Takasago International Corporation, Tokyo-to 144-8721 (JP)
(72) Inventor: ITOH, Hisanori, Hiratsuka-shi Kanagawa 254-0073 (JP); MAEDA, Hironori, Hiratsuka-shi Kanagawa 254-0073 (JP); HORI, Yoji, Hiratsuka-shi Kanagawa 254-0073 (JP)
(74) Representative: Uchida, Kenji
(86) International application number: PCT/JP2011/054980
(87) International publication number: WO 2011/108672

(57) **Abstract**

The present invention provides a catalyst for producing optically active aldehyde or optically active ketone, as an optically active carbonyl compound, by selectively asymmetrically hydrogenating an α, β-unsaturated carbonyl compound, and in particular, a catalyst insoluble in a reaction mixture for obtaining optically active citronellal useful as a flavoring by selectively asymmetrically hydrogenating citral, geranial or neral, and a method for producing the corresponding optically active carbonyl compound. In particular, the present invention provides a method for producing citronellol of identical optical isomers regardless of a mixing ratio of neral and geranial in the asymmetric hydrogenation of citral. In the present invention, the α, β-unsaturated carbonyl compound is asymmetrically hydrogenated using a catalyst including a complex of at least one transition metal selected from transition metals belonging to Group 8 to Group 10 of the Periodic Table, and an optically active cyclic nitrogen-containing compound, and an acid if necessary.

## Description

### Technical Field

The present invention relates to a method for producing an optically active aldehyde or optically active ketone, which is an optically active carbonyl compound, by selectively asymmetrically hydrogenating carbon-carbon double bonds of α,β-unsaturated carbonyl compounds using a catalyst for homogenous asymmetric hydrogenation.

### Background Art

Conventionally, there have been made an attempt for conducting asymmetric hydrogenation of carbon-carbon double bond of α,β-unsaturated aldehyde using hydrogen gas, and there is known a method for conducting asymmetric hydrogenation of neral or geranial for the purpose of obtaining optically active citronellal which is particularly important as a flavor or fragrance (Patent Documents 1 and 2). Since these methods are methods for hydrogenating carbon-carbon double bond with hydrogen gas using a small amount of a homogeneous catalyst, auxiliaries are not required so that a large amount of waste is not generated.

In addition, asymmetric hydrogation of carbon-carbon double bond of α,β-unsaturated keton using a homogeneous catalys has been reported (Patent Document 3, and Non-Patent Document 1).
On the other hand, there has been reported a hydrogen transfer type asymmetric hydrogenation reaction of an α,β-unsaturated compound using an organic asymmetric catalyst and Hantzsch ester (Patent Document 4, Non-Patent Document 2).

### Prior Art Documents

### Patent Documents

Patent Document 1: JP-B-61-23775
Patent Document 2: JP-A-2008-515843
Patent Document 3: JP-A-9-502459
Patent Document 4: US 2006/0161024

### Non-patent Document

Non-patent Document 1: J. Org. Chem. 1995, 60, P357
Non-patent Document 2: Acc. Chem. Res. 2007, 40, 1327-1339

### Summary of Invention

### Technical Problem

However, catalysts used for the methods of Patent Document 1 and 2 are known as catalysts for homogeneous hydrogenation that use expensive rhodium metals containing expensive optically active ligands. In the case of asymmetric hydrogenation of citral (mixture of geranial and neral) using the catalysts for homogeneous hydrogenation, neral and geranial should be separately subjected to the reaction, since different optical isomers are obtained from neral and geranial, respectively.
In addition, the methods of using organic catalyst described in Non-Patent Document 1 and Patent Document 3 are economically disadvantageous as a method for producing an optically active aldehyde or an optically active ketone, because a catalyst quantity of about 20% by mol based on the raw material unsaturated aldehyde or unsaturated ketone is required and the Hantzsch ester as the substrate of hydrogenation is required in an amount of equal to or larger than the raw material unsaturated aldehyde or unsaturated ketone.

An object of the present invention relates to a method for producing corresponding optically active aldehydes or optically active ketones by asymmetrically hydrogenating carbon-carbon double bonds of α,β-unsaturated carbonyl compounds using an organic catalyst and an inexpensive transition metal complex as catalysts for asymmetric hydrogenation. In particular, the present invention provides a method for producing optically active citronellal by hydrogenating citral, geranial or neral through asymmetric hydrogenation reaction.

### Means for Solving the Problems

As a result of intensely repeated research to solve these problems, the present inventors had discovered that the corresponding optically active aldehyde or optically active ketone can be obtained through asymmetric hydrogenation of α,β-unsaturated carbonyl compounds by using a specific transition metal complex, and optically active cyclic nitrogen-containing compound, or a specific transition metal complex, optically active cyclic nitrogen-containing compound and an acid. The present invention has beem completed based on this discovery.
In particular, the present inventors had discovered that identical optical isomers can be obtained from a single catalyst such as any one of neral and geranial which have relation between Z-configuration and E-configuration, or citral which is a mixture of neral and geranial, by using the catalyst system of the present invention
The catalyst of the present invention provides a completely new concept in that identical optical isomers can be prepared regardless of a mixing ratio of neral and geranial in the asymmetric hydrogenation of citral.

That is, the present invention encompasses each of the following inventions.
<1> A catalyst for homogenous asymmetric hydrogenation, comprising:
   a complex of at least one transition metal selected from transition metals belonging to Group 8 to Group 10 of the Periodic Table; and
   an optically active cyclic nitrogen-containing compound.
<2> The catalyst for homogenous asymmetric hydrogenation according to <1>, which is formed by coordinating the the optically active cyclic nitrogen-containing compound to the at least one transition metal selected from transition metals belonging to Group 8 to Group 10 of the Periodic Table.
<3> A catalyst for homogenous asymmetric hydrogenation, comprising:
   a complex of at least one transition metal selected from transition metals belonging to Group 8 to Group 10 of the Periodic Table;
   an optically active cyclic nitrogen-containing compound; and
   an acid.
<4> The catalyst for homogenous asymmetric hydrogenation according to any one of <1> to <3>, wherein the optically active cyclic nitrogen-containing compound is a compound represented by the general formula (1):

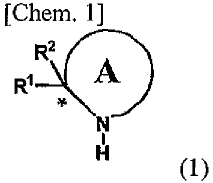

(wherein ring A is a 3- to 7-membered ring which may have a substituent group and comprises at least one kind of an atom selected from the group consisting of carbon, nitrogen, sulfur, oxygen, and phosphorous, and
R¹ and R² each independently represent a hydrogen atom, an alkyl group which may have a substituent group, a cycloalkyl group which may have a substituent group, an alkenyl group which may have a substituent group, an aryl group which may have a substituent group, an aralkyl group which may have a substituent group, an alkoxy group which may have a substituent group, a carboxyl group which may have a substituent group, an alkoxycarbonyl group which may have a substituent group, an amide group which may have a substituent group, a carbamoyl group which may have a substituent group, or a siloxy group which may have a substituent group, wherein R¹ and R² do not represent a same substituent group, and either R¹ or R² may be bonded to the ring A to form a ring; and * represents an asymmetric carbon atom).
<5> The catalyst for homogenous asymmetric hydrogenation according to any one of <1> to <4>, wherein the complex of the transition metal is a complex of a transition metal selected from nickel, ruthenium, rhodium, iridium, palladium and platinum.
<6> A manufacturing method of an optically active carbonyl compound, wherein an α,β-unsaturated carbonyl compound is reacted with hydrogen or a hydrogen-donating compound in the presence of the catalyst for homogenous asymmetric hydrogenation according to any one of claims 1 to 5, thereby manufacturing the optically active carbonyl compound.
<7> The manufacturing method according to <6>, wherein the α,β-unsaturated carbonyl compound is an α,β-unsaturated carbonyl compound represented by the general formula (2):

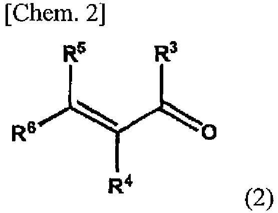

(wherein R³, R⁴, R⁵ and R⁶ each independently represent a hydrogen atom, an alkyl group which may have a substituent group, a cycloalkyl group which may have a substituent group, an alkenyl group which may have a substituent group, an aryl group which may have a substituent group, or an aralkyl group which may have a substituent group, wherein a ring may be formed by R⁵ and R⁶; when R⁴ does not represent a hydrogen atom, R⁵ and R⁶ may be the same; and when R⁴ represents a hydrogen atom, R⁵ and R⁶ do not represent a hydrogen atom and are different from each other); and
the prepared optically active carbonyl compound is an optically active carbonyl compound represented by the general formula (3):

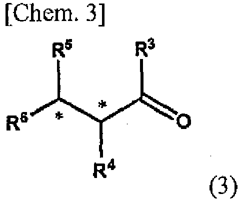

(wherein R³, R⁴, R⁵ and R⁶ are the same as defined in the formula (2), and two * mean that at least one * represents an asymmetric carbon atom).
<8> The manufacturing method according to <7>, wherein the α,β-unsaturated carbonyl compound is geranial, neral or citral.
<9> The manufacturing method according to <7>, wherein the α,β-unsaturated carbonyl compound is cyclic ketones having a 5- to 16-membered ring.
<10> A manufacturing method of an optically active carbonyl compound, wherein an α,β-unsaturated carbonyl compound is reacted with hydrogen or a hydrogen-donating compound in the presence of a complex of at least one transition metal selected from transition metals belonging to Group 8 to Group 10 of the Periodic Table and an optically active cyclic nitrogen-containing compound, thereby manufacturing the optically active carbonyl compound.
<11> A manufacturing method of an optically active carbonyl compound, wherein an α,β-unsaturated carbonyl compound is reacted with hydrogen or a hydrogen-donating compound in the presence of a complex of at least one transition metal selected from transition metals belonging to Group 8 to Group 10 of the Periodic Table, an optically active cyclic nitrogen-containing compound and an acid, thereby manufacturing the optically active carbonyl compound.
<12> The manufacturing method according to <10> or <11>, wherein the optically active cyclic nitrogen-containing compound is a compound represented by the general formula (1):

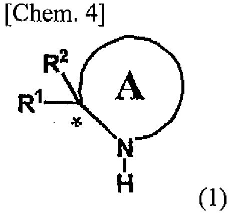

(wherein ring A is a 3- to 7-membered ring which may have a substituent group and comprises at least one kind of an atom selected from the group consisting of carbon, nitrogen, sulfur, oxygen, and phosphorous; and
R¹ and R² each independently represent a hydrogen atom, an alkyl group which may have a substituent group, a cycloalkyl group which may have a substituent group, an alkenyl group which may have a substituent group, an aryl group which may have a substituent group, an aralkyl group which may have a substituent group, an alkoxy group which may have a substituent group, a carboxyl group which may have a substituent group, an alkoxycarbonyl group which may have a substituent group, an amide group which may have a substituent group, a carbamoyl group which may have a substituent group, or a siloxy group which may have a substituent group, wherein R¹ and R² do not represent a same substituent group, and either R¹ or R² may be bonded to the ring A to form a ring; and * represents an asymmetric carbon atom).
<13> The manufacturing method according to any one of <10> to <12>, wherein the optically active cyclic nitrogen-containing compound is a compound represented by the general formula (1):

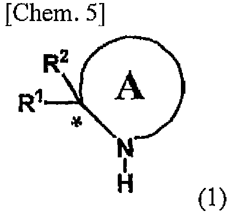

(wherein ring A is a 3- to 7-membered ring which may have a substituent group and comprises at least one atom selected from the group consisting of carbon, nitrogen, sulfur, oxygen, and phosphorous; and
R¹ and R² each independently represent a hydrogen atom, an alkyl group which may have a substituent group, a cycloalkyl group which may have a substituent group, an alkenyl group which may have a substituent group, an aryl group which may have a substituent group, an aralkyl group which may have a substituent group, an alkoxy group which may have a substituent group, a carboxyl group which may have a substituent group, an alkoxycarbonyl group which may have a substituent group, an amide group which may have a substituent group, a carbamoyl group which may have a substituent group, or a siloxy group which may have a substituent group, wherein R¹ and R² do not represent a same substitutent, and either R¹ or R² may be bonded to the ring A to form a ring; and * represents an asymmetric carbon atom).
<14> The manufacturing method according to any one of <10> to <13>, wherein the complex of the transition metal is a complex of a transition metal selected from nickel, ruthenium, rhodium, iridium, palladium and platinum.
<15> The manufacturing method according to any one of <10> to <14>, wherein the α,β-unsaturated carbonyl compound is a compound represented by the general formula (2):

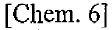

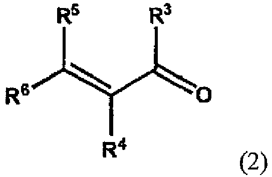

(wherein R³, R⁴, R⁵ and R⁶ each independently represent a hydrogen atom, an alkyl group which may have a substituent group, a cycloalkyl group which may have a substituent group, an alkenyl group which may have a substituent group, an aryl group which may have a substituent group, or an aralkyl group which may have a substituent group, wherein a ring may be formed by R⁵ and R⁶; when R⁴ does not represent a hydrogen atom, R⁵ and R⁶ may be the same; and when R⁴ represents a hydrogen atom, R⁵ and R⁶ do not represent a hydrogen atom and are different from each other), and
the prepared optically active carbonyl compound is an optically active carbonyl compound represented by the general formula (3):

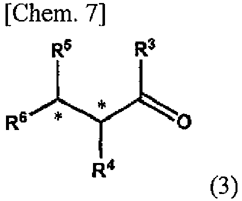

(wherein R³, R⁴, R⁵ and R⁶ are the same as defined in the formula (2), and two * mean that at least one * represents an asymmetric carbon atom).
<16> The manufacturing method according to <15>, wherein the α,β-unsaturated carbonyl compound is geranial, neral or citral.
<17> The manufacturing method according to <15>, wherein the α,β-unsaturated carbonyl compound is cyclic ketones having a 5- to 16-membered ring.

### Effects of Invention

As described above, in the present invention, as a catalyst for asymmetric hydrogenation reaction, a transition metal complex and an optically active cyclic nitrogen-containing compound as an additive contributing to enantioselectivity are used, or a transition metal complex, an optically active cyclic nitrogen-containing compound as an additive contributing to enantioselectivity and an acid are used.
The catalyst for asymmetric hydrogenation of the present invention may be previously prepared and then subjected to reaction as in conventional catalysts for asymmetric hydrogenation, or the reaction for previously preparing the catalyst may be unnecessary. That is, in the asymmetric hydrogenation reaction of the present invention, asymmetric hydrogenation can be performed by simply mixing a starting compound, an optically active cyclic nitrogen-containing compounds and a transition metal complex, and further adding an acid thereto if necessary. As such, the present invention is industrially advantageous in that operations are simple, the transition metal complex and the optically active cyclic nitrogen-containing compound can be recovered to recycle.

In addition, when the catalyst of the present invention is used, although any one of Z- and E-configuration compounds is used for the substrate at the double bonds of α-position and β-position of α,β-unsaturated carbonyl compounds, the steric configuration of the produced optically active carbonyl compound depends the steric configuration of used optically active cyclic nitrogen-containing compound, not steric configuration of the substrate. Accordingly, according to the present invention, although a mixture of a Z configuration compound and an E configuration compound is used as a substrate, the optically active carbonyl compound of identical steric configuration can be prepared.

### Brief description of the drawings

Fig.1 is an H¹-NMR chart of a complex (palladium acetate + (R)-(+)-2-(diphenylmethyl) pyrrolidine) in Example 2-1.
Fig. 2 is an enlarged view of a low magnetic field part of the H¹-NMR chart of Fig.1.
Fig. 3 is an H¹-NMR chart of a complex of Example 2-2 (palladium acetate+(R)-(+)-2-(diphenylmethyl) pyrrolidine + trifluoro acetic acid).
Fig. 4 is an enlarged view of a low magnetic field part of the H¹-NMR chart of Fig. 3.
Fig. 5 is an H¹-NMR chart of (R)-(+)-2-(diphenylmethyl)pyrrolidine.
Fig. 6 is an enlarged view of a low magnetic field part of the H¹-NMR chart of Fig. 5.
Fig. 7 is an H¹-NMR chart of (R)-(+)-2-(diphenylmethyl)pyrrolidine + trifluoroacetic acid.
Fig. 8 is an enlarged view of a low magnetic field part of the H¹-NMR chart of Fig. 7.

### Mode for Carrying Out the Invention

The following describes the present invention in detail.

According to the present invention, an α,β-unsaturated carbonyl compound is used as the substrate, and an optically active aldehyde or an optically active ketone, which is an optically active carbonyl compound, is produced by subjecting this to asymmetric hydrogenation using the catalyst of the present invention.

### <Substrate>

As the α,β-unsaturated carbonyl compound to be used as the substrate, a compound represented by the following general formula (2) can, for example, be mentioned, though not particularly limited thereto. In this connection, in the case of the presence of Z-configuration and E-configuration regarding the α-position and β-position double bond of the α,β-unsaturated carbonyl compound, all of them are included therein.

The following general formula (2):

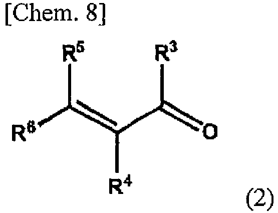

In the formula, R³, R⁴, R⁵ and R⁶ each independently represent a hydrogen atom, an alkyl group which may have a substituent group, a cycloalkyl group which may have a substituent group, an alkenyl group which may have a substituent group, an aryl group which may have a substituent group, or an aralkyl group which may have a substituent group, wherein a ring may be formed by R⁵ and R⁶; when R⁴ does not represent a hydrogen atom, R⁵ and R⁶ may be the same; and when R⁴ represents a hydrogen atom, R⁵ and R⁶ do not represent a hydrogen atom and are different from each other. That is, there is no case in which all of R⁴, R⁵ and R⁶ present a hydrogen atom.

An optically active aldehyde or an optically active ketone, which is an optically active carbonyl compound represented by the following formula (3), is produced by subjecting a compound represented by the aforementioned formula (2) to asymmetric hydrogenation using the catalyst of the present invention.

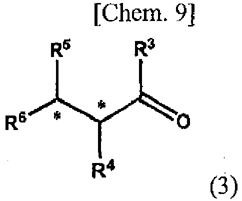

In the formula, R³, R⁴, R⁵ and R⁶ are the same as defined in the formula (2), and two * mean that one or both of them represent an asymmetric carbon atom.

Regarding the α,β-unsaturated carbonyl compound represented by the general formula (2) and the optically active carbonyl compound represented by the general formula (3), the groups represented by R³, R⁴, R⁵ and R⁶, namely the alkyl group, cycloalkyl group, alkenyl group, aryl group, aralkyl group, alkoxycarbonyl group, carbonyloxy group, nitrile group and perhalogenoalkyl group are described. Each of these groups may have a substituent group.

As the alkyl group, examples thereof include a straight chain or branched chain alkyl group, for example, having from 1 to 30 carbon atoms, preferably from 1 to 10 carbon atoms, and illustrative examples thereof include methyl group, ethyl group, n-propyl group, isopropyl group, n-butyl group, 2-butyl group, isobutyl group, tert-butyl group, n-pentyl group, 2-pentyl group, 3-pentyl group, tert-pentyl group, 2-methylbutyl group, 3-methylbutyl group, 2,2-dimethylpropyl group, 1,2-dimethylpropyl group, n-hexyl group, 2-hexyl group, 3-hexyl group, 2-methylpentyl group, 3-methylpentyl group, 4-methylpentyl group, 1,1-dimethylbutyl group, 1,2-dimethylbutyl group, 1,3-dimethylbutyl group, 2,2-dimethylbutyl group, 2,3-dimethylbutyl group, 3,3-dimethylbutyl group, 2-ethylbutyl group, 1,1,2-trimethylpropyl group, 1,2,2-trimethylpropyl group, 1-ethyl-1-methylpropyl group, 1-ethyl-2-methylpropyl group, heptyl group, octyl group, nonyl group, decyl group, undecyl group, dodecyl group, tridecyl group, tetradecyl group, pentadecyl group, hexadecyl group, heptadecyl group, octadecyl group, nonadecyl group, eicosyl group, heneicosyl group, docosyl group and the like.

In addition, these alkyl groups may have a substituent group, and as said substituent group of alkyl groups, examples thereof include an alkenyl group, an alkynyl group, an aryl group, an aliphatic heterocyclic group, an aromatic heterocyclic group, an alkoxy group, an alkylenedioxy group, an aryloxy group, an aralkyloxy group, a heteroaryloxy group, a substituted amino group, nitro group, nitrile group, a perhalogenoalkyl group, a halogen atom and the like.

As the alkenyl group as a substituent group of alkyl group, examples thereof include a straight chain or branched chain alkenyl group having, for example, from 2 to 20 carbon atoms, preferably from 2 to 10 carbon atoms, more preferably from 2 to 6 carbon atoms, and illustrative examples thereof include vinyl group, propenyl group, 1-butenyl group, pentenyl group, hexenyl group and the like.

As the alkynyl group as a substitent group of alkyl group, examples thereof include a straight chain or branched chain alkynyl group having, for example, from 2 to 15 carbon atoms, preferably from 2 to 10 carbon atoms, more preferably from 2 to 6 carbon atoms, and illustrative examples thereof include, ethynyl group, 1-propynyl group, 2-propynyl group, 1-butynyl group, 3-butynyl group, pentinyl group, hexynyl group and the like.

As the aryl group as a substituent group of alkyl group, examples thereof include an aryl group having, for example, from 6 to 14 carbon atoms, and illustrative examples thereof include, phenyl group, naphthyl group, anthryl group, phenanthryl group, biphenyl group, tolyl group, xylyl group, mesityl group, methoxyphenyl group, dimethoxyphenyl group, fluorophenyl group and the like.

As the aliphatic heterocyclic group as a substituent group of alkyl group, examples thereof include a group which has, for example, from 2 to 14 carbon atoms and contains, as heterogeneous atoms, at least one, preferably from 1 to 3 hetero atoms such as nitrogen atom, oxygen atom and sulfur atom. Preferably, a 5- or 6-membered monocyclic aliphatic heterocyclic group and a polycyclic or condensed ring aliphatic heterocyclic group can be mentioned. Illustrative examples of the aliphatic heterocyclic group include 2-oxo-1-pyrrolidinyl group, piperidino group, piperazinyl group, morpholino group, tetrahydrofuryl group, tetrahydropyranyl group, tetrahydrothienyl group and the like.

As the aromatic heterocyclic group as a substituent group of alkyl group, examples thereof include a group which has, for example, from 2 to 15 carbon atoms and contains, heterogeneous atoms, at least one, preferably from 1 to 3 hetero atoms such as nitrogen atom, oxygen atom and sulfur atom. Preferably, a 5- or 6-membered monocyclic aromatic heterocyclic group and a polycyclic or condensed ring aromatic heterocyclic group can be mentioned. Illustrative examples of the aromatic heterocyclic group include furyl group, thienyl group, pyridyl group, pyrimidinyl group, pyrazinyl group, pyridazinyl group, pyrazolinyl group, imidazolyl group, oxazolinyl group, thiazolinyl group, benzofuryl group, benzothienyl group, quinolyl group, isoquinolyl group, quinoxalinyl group, phtharazinyl group, quinazolinyl group, naphthylidinyl group, cinnolinyl group, benzimidazolyl group, benzoxazolyl group, benzothiazolyl group and the like.

As the alkoxy group as a substituent group of alkyl group, examples thereof include a straight chain or branched chain alkoxy group having, for example, from 1 to 6 carbon atoms, and illustrative examples thereof include methoxy group, ethoxy group, n-propoxy group, isopropoxy group, n-butoxy group, 2-butoxy group, isobutoxy group, tert-butoxy group, n-pentyloxy group, 2-methylbutoxy group, 3-methylbutoxy group, 2,2-dimethylpropoxy group, n-hexyloxy group, 2-methylpentyloxy group, 3-methylpentyloxy group, 4-methylpentyloxy group, 5-methylpentyloxy group and the like.

As the alkylenedioxy group as a substituent group of alkyl group, examples thereof include an alkylenedioxy group having, for example, from 1 to 3 carbon atoms, and illustrative examples thereof include methylenedioxy group, ethylenedioxy group, propylenedioxy group, isopropylidenedioxy group and the like.

As the aryloxy group as a substituent group of alkyl group, examples thereof include an aryloxy group having, for example, from 6 to 14 carbon atoms, and illustrative examples thereof include phenoxy group, naphthyloxy group, anthryloxy group and the like.

As the aralkyloxy group as a substituent group of alkyl group, examples thereof include an aralkyloxy group having from 7 to 12 carbon atom, and illustrative examples thereof include benzyloxy group, 2-phenylethoxy group, 1-phenylpropoxy group, 2-phenylpropoxy group, 3-phenylpropoxy group, 1-phenylbutoxy group, 2-phenylbutoxy group, 3-phenylbutoxy group, 4-phenylbutoxy group, 1-phenylpentyloxy group, 2-phenylpentyloxy group, 3-phenylpentyloxy group, 4-phenylpentyloxy group, 5-phenylpentyloxy group, 1-phenylhexyloxy group, 2-phenylhexyloxy group, 3-phenylhexyloxy group, 4-phenylhexyloxy group, 5-phenylhexyloxy group, 6-phenylhexyloxy group and the like.

As the heteroaryloxy group as a substituent group of alkyl group, examples thereof include a heteroaryloxy group which has, for example, from 2 to 14 carbon atoms and contains, as heterogeneous atoms, at least one, preferably from 1 to 3 hetero atoms such as nitrogen atom, oxygen atom and sulfur atom, and illustrative examples thereof include 2-pyridyloxy group, 2-pyrazyloxy group, 2-pyrimidyloxy group, 2-quinolyloxy group and the like.

As the substituted amino group as a substituent group of alkyl group, examples thereof include mono- or di-alkylamino groups such as N-methylamino group, N,N-dimethylamino group, N,N-diethylamino group, N,N-diisopropylamino group and N-cyclohexylamino group; mono- or di-arylamino group such as N-phenylamino group, N,N-diphenylamino group, N-naphthylamino group and N-naphthyl-N-phenylamino group; mono- or di-aralkylamino group such as N-benzylamino group and N,N-dibenzylamino group.

Examples of perhalogenoalkyl group substituting an alkyl group include a trifluoromethyl group, a pentafluoroethyl group, a heptafluoropropyl group, a nonafluorobutyl group, a trichloromethyl group, a pentachloroethyl group and the like.

As the halogen atom substituting the alkyl group, examples thereof include fluorine atom, chlorine atom, bromine atom, iodine atom and the like.

As the cycloalkyl group, examples thereof include cyclopropyl group, cyclobutyl group, cyclopentyl group, cyclohexyl group and the like.
These cycloalkyl groups may have a substituent group, and as said substituent group, the substituent groups described in the aforementioned description on the substituent group of alkyl group can be mentioned.

As the alkenyl group, examples thereof include a straight or branched chain or cyclic alkenyl group having, for example, from 2 to 20 carbon atoms, preferably from 2 to 10 carbon atoms. Illustrative examples thereof include alkenyl groups, for example, vinyl group, 1-propenyl group, 2-propenyl group, 1-butenyl group, 2-butenyl group, 3-butenyl group, 1-pentenyl group, 2-pentenyl group, 3-pentenyl group, 4-pentenyl group, 1-cyclopentenyl group, 3-cyclopentenyl group, 1-hexenyl group, 2-hexenyl group, 3-hexenyl group, 4-hexenyl group, 5-hexenyl group, 4-methyl-3-pentenyl group, 4,8-dimethyl-3,7-nonadienyl group, 1-cyclohexenyl group, 3-cyclohexenyl group and the like.
These alkenyl groups may have a substituent group, and as said substituent group, the groups described in the aforementioned description on the substituent group of alkyl group can be mentioned.

As the aryl group, examples thereof include an aryl group having, for example, from 6 to 14 carbon atoms, and illustrative examples thereof include phenyl group, naphthyl group, anthryl group, phenanthryl group, biphenyl group and the like. These aryl groups may have a substituent group, and as said substituent group, the groups described in the description on the substituent group of alkyl group can be mentioned.

As the aralkyl group, an aralkyl group having, for example, from 7 to 12 carbon atoms is desirable, and illustrative examples thereof include benzyl group, 2-phenylethyl group, 1-phenylpropyl group, 3-naphthylpropyl group and the like.
These aralkyl groups may have a substituent group, and as said substituent group, the groups described in the description on the substituent group of alkyl group can be mentioned.
As the alkoxycarbonyl group, an alkoxycarbonyl group having 2 to 15 carbon atoms is preferable, and examples thereof include a methoxycarbonyl group, an ethoxycarbonyl group, a propoxycarbonyl group, a butoxycarbonyl group, an isopropoxycarbonyl group, a phenoxycarbonyl group, a naphthoxycarbonyl group, a benzyloxycarbonyl group and the like.
These alkoxycarbonyl groups may have a substituent group and examples of the substituent group are the same as those mentioned in the description of the alkyl group.
As the acyloxy group, an aralkyl group having 2 to 15 carbon atoms is preferable, and examples thereof include an acetyloxy group, a propanoyloxy group, a butanoyloxy group, an octanoyloxy group, a benzoyloxy group, a toluoyloxy group, a xyloyloxy group, a naphthoyloxy group, a phenanthroyloxy group, an anthroyloxy group and the like.
These acyloxy groups may have a substituent group and examples of the substituent group are the same as those mentioned in the description of the alkyl group.

Regarding the ring formed by R³ and R⁴, R³ and R⁵, R³ and R⁶, R⁴ and R⁶ or R⁵ and R⁶ in the α,β-unsaturated carbonyl compound represented by the general formula (2) and the optically active carbonyl compound represented by the general formula (3), for example, there may be mentioned cyclopentane ring, cyclohexane ring, indane ring, tetralin ring, cyclopentene ring, cyclohexene ring, cycloheptene ring, indene ring, dihydronaphthalene ring, octahydronaphthalene ring, decahydronaphthalene ring and the like. These rings may be substituted with the aforementioned alkyl group or the acyl group described in the following.

As the acyl group, examples thereof include acetyl group, propanoyl group, butanoyl group, octanoyl group, benzoyl group, toluoyl group, xyloyl group, naphthoyl group, phenanthroyl group, anthroyl group and the like.

As illustrative examples of the α,β-unsaturated aldehyde to be used as the substrate in the present invention, for example, the following compounds can be mentioned. In this connection, in the case of the presence of Z-configuration and E-configuration regarding the α-position and β-position double bond of the α,β-unsaturated aldehyde, all of them are included therein. The wavy line in the following compounds represents Z-configuration and E-configuration or a mixture thereof.

In the following compounds, Me represents methyl group, Ph represents phenyl group, and Bn represents benzyl group.

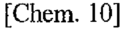

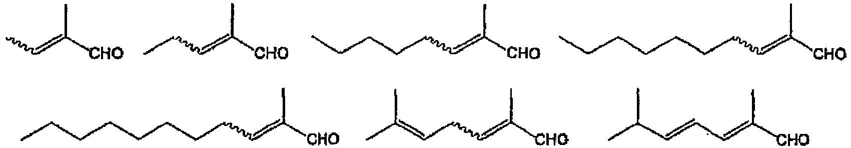

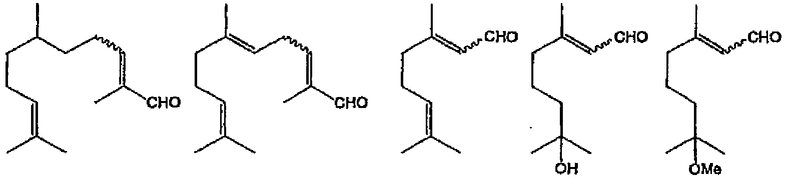

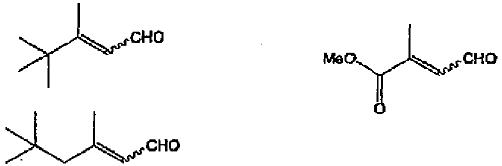

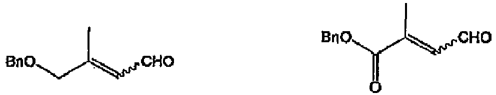

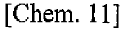

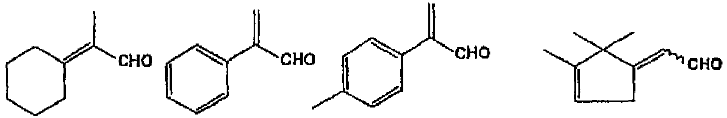

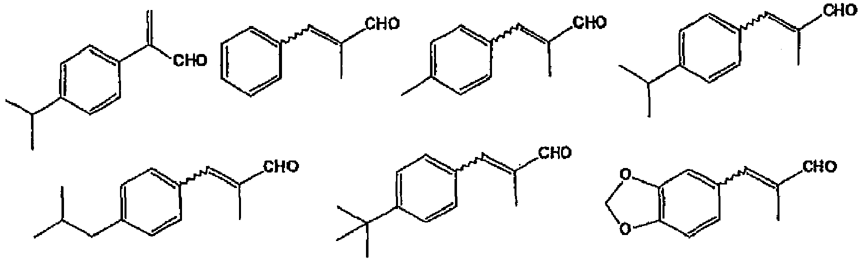

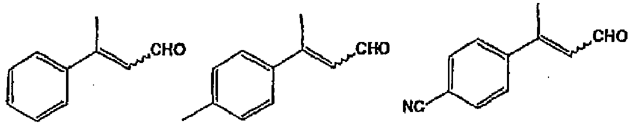

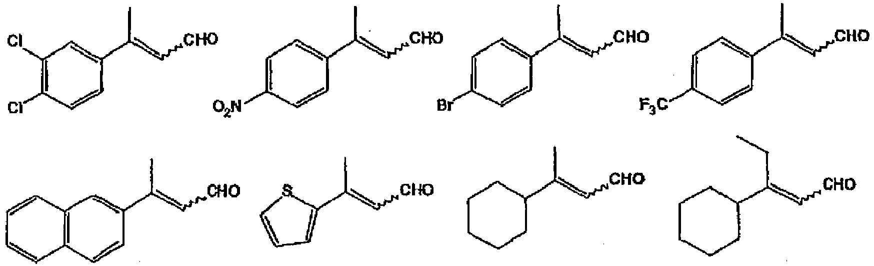

Among the aforementioned α,β-unsaturated aldehydes, geranial (the following A), neral (the following B) and citral can be mentioned as particularly desirable compounds.

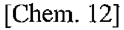

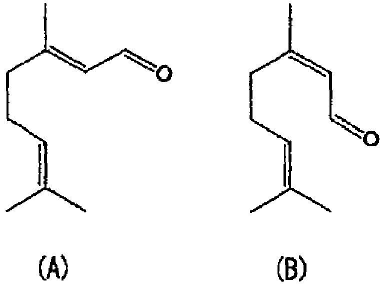

As the α,β-unsaturated ketone to be used as the substrate in the present invention, 5- to 16-membered ketones are desirable.
As illustrative examples of the α,β-unsaturated ketone, the following compounds can, for example, be mentioned. In this connection, in the case of the presence of Z-configuration and E-configuration regarding the α-position and β-position double bond of the α,β-unsaturated ketone, all of them are included therein. The wavy line in the following compounds represents Z-configuration and E-configuration or a mixture thereof.
In the following compounds, Ph represents phenyl group, Et represents ethyl group, Bu represents butyl group, Pr represents propyl group and Bn represents benzyl group.

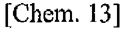

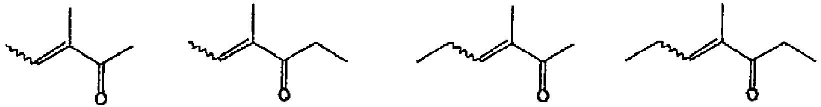

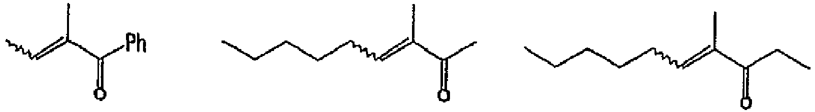

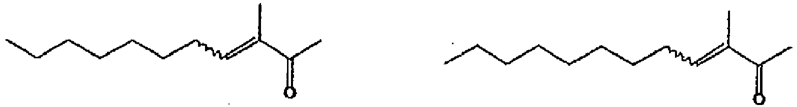

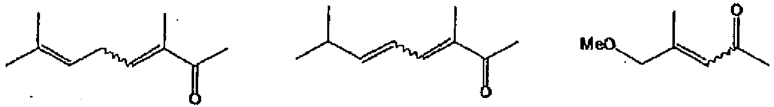

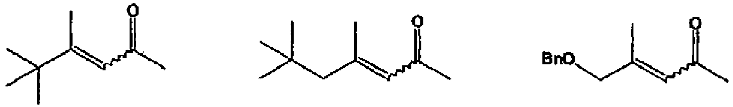

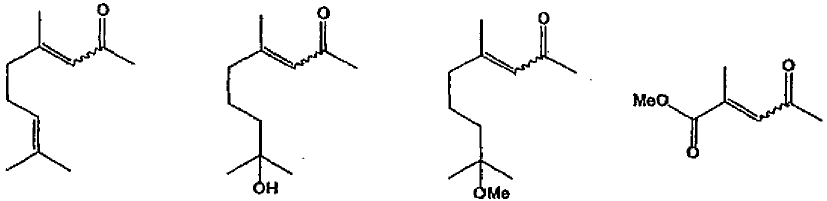

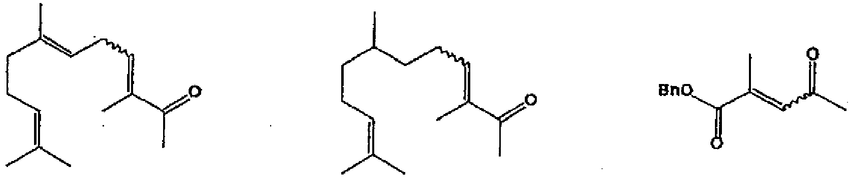

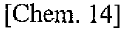

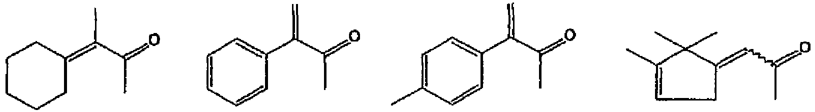

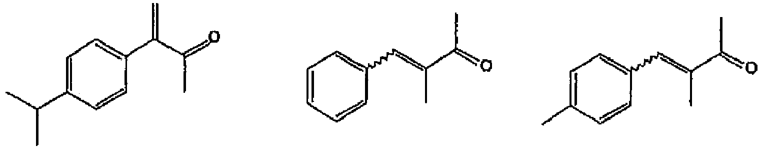

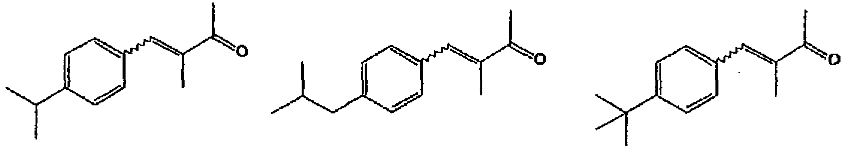

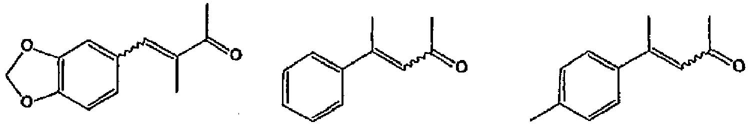

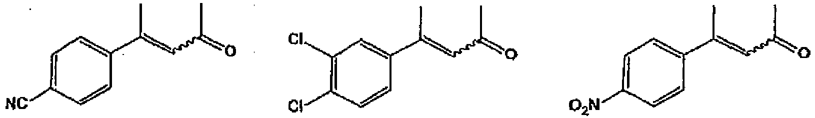

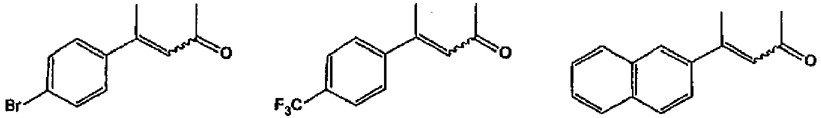

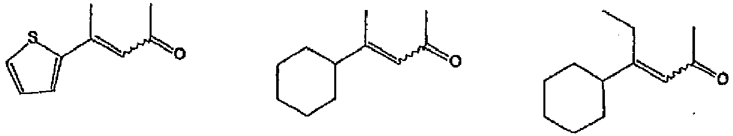

### <Catalyst>

Next, the catalyst of the present invention will be described.
The catalyst of the present invention contains a complex of at least one transition metal selected from transition metals belonging to Group 8 to Group 10 of the Periodic Table, an optically active cyclic nitrogen-containing compound, and an acid if necessary, and is a catalyst for homogenous asymmetric hydrogenation obtained by reacting these components.

The transition metal complex used for the present invention is a complex of at least one transition metal selected from transition metals belonging to Group 8 to Group 10 of the Periodic Table and is particularly not limited so long as it allows asymmetric hydrogenation reaction.
Examples of the metal belonging to Group 8 to Group 10 of the Periodic Table include Ni (nickel), Ru (ruthenium), Rh (rhodium), Ir (iridium), Pd (palladium) and Pt (platinum) and the metal is particularly preferably palladium. In addition, the valence of the complex is preferably 0 to 3 valances.

Examples of the ligand of the used metal complex include a hydrogen atom, an alkyl group, an alkoxy group, olefin, alkyne, π-allyl, an aryl group, carbene, nitrene, a halogen atom, a carboxyl group, carbon monoxide, isonitrile, monodentate to tridentate ligands such as nitrogen ligands and phosphorous ligands, and solvent ligands such as nitrile and tetrahydrofuran. A plurality of types of ligands may be coordinated and these ligands may be racemic substances or optically active substances.

Examples of the transition metal complex used for the present invention include compounds represented by the following general formula (4).

[MₘLₙWₚU_{q}]ᵣZₛ (4)

(wherein M represents a transition metal belonging to Group 8 to Group 10 of the Periodic Table; L represents a ligand; W represents a hydrogen atom, a halogen atom, an alkyl group, an aryl group, an alkoxy group, a carboxyl group, a diene or an anion; U represents a hydrogen atom, a halogen atom, an alkyl group, an alkoxy group, an aryl group, a carboxyl group, a diene, an anion, or a ligand other than L; Z represents an anion or amine; m and r represent an integer of 1 to 5; and n, p, q and s represent an integer of 0 to 5, and p+q+s is 1 or more)

In the general formula, examples of the transition metal belonging to Group 8 to Group 10 of the Periodic Table represented by M include Ni (nickel), Ru (ruthenium), Rh (rhodium), Ir (iridium), Pd (palladium), Pt (platinum) and the like.

Examples of the ligand represented by L include monodentate to tridentate ligands such as nitrogen ligands and phosphorous ligands.

Examples of halogen atom represented by W and U include a fluorine atom, a chlorine atom, a bromine atom, an iodine atom and the like.

Examples of the alkyl group represented by W and U include a methyl group, an ethyl group, an n-propyl group, an isopropyl group, an n-butyl group, an isobutyl group, an s-butyl group, a t-butyl group, a pentyl group, a hexyl group, a heptyl group, an octyl group and the like.

Examples of the aryl group represented by W and U include aromatic monocyclic or polycyclic groups such as a phenyl group, a naphthyl group, an anthryl group, a phenanthryl group, an indenyl group and a mesityl group.

Examples of the alkoxy group represented by W and U include a methoxy group, an ethoxy group, a propoxy group, an isopropoxy group, an n-butoxy group, a t-butoxy group, a phenoxy group, a benzyloxy group and the like.

Examples of the carboxyl group represented by W and U include a formyloxy group, an acetoxy group, a propionyloxy group, a butyryloxy group, a benzoyl group and the like.

Examples of the diene represented by W and U include butadiene, 1,5-cyclooctadiene, norbornadiene and the like.

Examples of the anion represented by W and U include NO₃, SO₄, CO₃, BH₄, BH₄, I₃, ClO₄, OTf (in which Tf represents a triflate group (SO₃CF₃)), PF₆, SbF₆, BPh₄(in which Ph represents a phenyl group) and the like.

Examples of the ligand other than L represented by U include aromatic compounds or olefin such as neutral ligands, alkylnitrile having 1 to 5 carbon atoms, benzonitrile, phthalonitrile, pyridine or substituted pyridine, dimethylsulfoxide, dimethylformamide, dimethylacetamide, acetone, dibenzylidene acetone (dba) and the like.

Examples of the anion represented by Z include NO₃, SO₄, CO₃, BH₄, BH₄, I₃, ClO₄, OTf, PF₆, SbF₆, BPh₄ and the like.

Examples of the amine represented by Z include trialkylamine compounds, diamine compounds, pyridines, dialkyl ammonium ions and the like.

Specific examples of the transition metal complex include nickel complexes such as bis(1,5-cyclooctadiene)nickel, 1,2-bis(diphenylphosphino)ethane nickel, bis(triphenylphosphine)nickel bromide, bis(triphenylphosphine)nickel chloride, bis(triphenylphosphine)nickel dicarbonyl, methallyl nickel chloride dimers, nickel acetate, nickel acetoacetonate, and nickel trifluoromethanesulfonate.

Examples of the ruthenium complex include bis(2-methylallyl)(1,5-cyclooctadiene)ruthenium, carbonyl chlorohydride tris(triphenylphosphine)ruthenium, carbonyl(dihydride)tris(triphenylphosphine)ruthenium, chloro(cyclopentadienyl)bis(triphenylphosphine)ruthenium, cyclopentadienyl(paracymene)ruthenium hexafluorophosphate, dichloro(paracymene)rutheniumdimer, and dichloro(1,5-cyclooctadiene) ruthenium polymers.

Examples of the rhodium complex include acetylacetonate(1,5-cyclooctadiene)rhodium, bis(1,5-cyclooctadiene)rhodium tetrafluoroborate, bis(1,5-cyclooctadiene)rhodium trifluoromethanesulfonate, bis(norbornadiene)rhodium tetrafluoroborate, chlorocarbonyl bis(triphenylphosphine)rhodium, chloro(1,5-cyclooctadiene)rhodium dimers, dicarbonyl acetyl acetonate rhodium, rhodium aceate dimers, hexarhodiumhexadecacarbonyl, and chlorotris(triphenylphosphine)rhodium.

Examples of the iridium complex include chlorocarbonyl bis(triphenylphosphine)iridium dimers, chloro(1,5-cyclooctadiene)iridium dimers, 1,5-cyclooctadiene(acetylacetonate)iridium, dicarbonyl acetylacetonate iridium, dichloro(pentamethylcyclopentadienyl)iridium dimers, hydride carbonyl tris(triphenylphosphine)iridium, iridium acetylacetonate, and trichloroiridium.

Examples of the palladium complex include allyl palladium chloride dimers, bis[1,2-bis(diphenylphosphino)ethane] palladium, tris(dibenzylideneacetone)di palladium, palladium chloride, bis(2-methylallyl)palladium chloride dimers, diacetatebis(triphenylphosphine)palladium, dichlorobis(acetonitrile) palladium, dichloro[1,2-bis(diphenylphosphino)ethane]palladium, trans-dichlorobis(triphenylphosphine) palladium, dichloro(1,5-cyclooctadiene)palladium, cis-dichloro(N,N,N',N'-tetramethylethylenediamine) palladium, palladium acetate, palladium acetylacetonate, and tetrakis(triphenylphosphine) palladium.

Examples of the platinum complex include bis(ethylenediamine)platinum, bis(tri-t-butylphosphine)platinum, dibromo(1,5-cyclooctadiene)platinum, dichlorobis(benzonitrile)platinum, cis-dichlorobis(pyridine)platinum, cis-(dichlorobis)triphenylphosphineplatinum, dichloro(1,5-cyclooctadiene)platinum, dichloro(dicyclopentadienyl)platinum, platinum acetylacetonate, platinum chloride, and tetrakis(triphenylphosphine)platinum.

The catalyst of the present invention may include an auxiliary ligand. Accordingly, the reactivity of the complex catalyst can be improved and selectivity of asymmetric hydrogenation can be improved.
Specifically, preferred examples thereof include carbon monoxide, cyclopentadiene, pentamethylcyclopentadiene, cyclooctene, cyclooctadiene (COD), p-cymene, acetonitrile, benzonitrile, picolinic acid, triphenylphosphine, tri-t-butylphosphine, triphenylphosphite, triphenylphosphate, tricyclohexylphosphine, di-t-butyl(2,2-diphenyl-1-methyl-1-cyclopropyl)phosphine (BRIDP), tri-n-octylphosphine, 2,2'-bis(diphenylphosphino)-1, 1'-binaphthyl (BINAP), 2,2'-bis(5,5',6,6',7,7',8,8'-octahydrodiphenylphosphino)-1, 1'-binaphthyl (H8-BINAP), 5,5'-bis(diphenylphosphino)-4,4'-bi-1,3-benzoxazole (SEGPHOS), 2,2'-bis(diphenylphosphino)-1,1'-biphenyl (BIPHEP), 1,2-bis((2R,5R)-2,5-dimethyl-phosphorano)benzene (DUPHOS), bis(diphenylphosphino)buthane (Chiraphos), 1,2-bis(diphenylphosphino)ethane (Dppe), 1,2-bis(diphenylphosphino)ferrocene (Dppf), 1,2-bis(diphenylphosphino)propane (Dppp), 2,2'-bipyridine, 2,2' : 6',2"-ter-pyridine, N-methylprolineethylester, and N-methyl-2-diphenylmethyl pyrrolidine. The auxiliary ligand may be an optically active substance.
The auxiliary ligand is preferably included in an amount of 0.5 to 8 equivalents with respect to the metal atom. Within this content, performance of the metal complex catalyst can be improved according to the corresponding metal atom.

Next, the optically active cyclic nitrogen-containing compound used as a catalyst component of the present invention will be described. In the catalyst of the present invention, the optically active cyclic nitrogen-containing compound is coordinated to a transition metal belonging to Group 8 to Group 10 of the Periodic Table, as a catalyst metal.
In the present invention, steric configuration of the optically active carbonyl compound, as the target product, depends on steric configuration of the optically active cyclic nitrogen-containing compound used as a cocatalyst component, rather than steric configuration of a substrate. Accordingly, in the present invention although the α,β-unsaturated carbonyl compounds serving as the substrate uses any one of a Z-configuration compound and an E-configuration compound, and uses a mixture thereof as a substrate, an optically active carbonyl compound having an identical steric configuration can be prepared.

As the optically active cyclic nitrogen-containing compound, for example, an optically active cyclic nitrogen-containing compound represented by the general formula (1) can be mentioned.

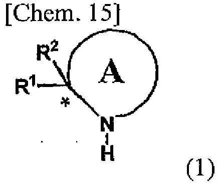

In the formula, ring A is a 3- to 7-membered ring which may have a substituent group and includes at least one kind of an atom selected from the group consisting of carbon, nitrogen, sulfur, oxygen, and phosphorous; and
R¹ and R² each independently represent a hydrogen atom, an alkyl group which may have a substituent group, a cycloalkyl group which may have a substituent group, an alkenyl group which may have a substituent group, an aryl group which may have a substituent group, an aralkyl group which may have a substituent group, an alkoxy group which may have a substituent group, a carboxyl group which may have a substituent group, an alkoxycarbonyl group which may have a substituent group, an amide group which may have a substituent group, a carbamoyl group which may have a substituent group, or a siloxy group which may have a substituent group, wherein R¹ and R² do not represent a same substituent group, and either R¹ or R² may be bonded to the ring A to form a ring; and * represents an asymmetric carbon atom.

As basic skeleton of the ring A, for example, aziridine skeleton, azetidine skeleton, pyrrolidine skeleton, indoline skeleton, pyrroline skeleton, pyrazolidine skeleton, imidazolidine skeleton, imidazolidinone skeleton, pyrazoline skeleton, thiazolidine skeleton, piperidine skeleton, piperazine skeleton, morpholine skeleton, thiomorpholine skeleton and the like can be mentioned. A substituent group may be present in these basic skeletons.

Examples of the substituent group present in the basic skeleton of the ring A include an oxo group, a halogen group, an alkyl group, an alkoxy group, an amino group, a hydroxycarbonyl group, an alkoxycarbonyl group, an acyl group, an aryl group, and an aralkyl group. Examples of the alkyl group, alkoxy group, alkoxycarbonyl group, aryl group and aralkyl group are the same as those mentioned in the description of R¹ and R². Examples of the halogen atom include a fluorine atom, a chlorine atom, a bromine atom and an iodine atom. Examples of the amino group include an amino group, a methylamino group, an ethylamino group, a propylamino group, an isopropylamino group, a butylamino group, a t-butylamino group, an octylamino group, a cyclohexylamino group, a phenylamino group, a styrenylamino group, a naphthylamino group, a benzylamino group, an anthracenylamino group, a dimethylamino group, a diethylamino group, a dipropylamino group, a diisopropylamino group, a dibutylamino group, a di-t-butylamino group, a dioctylamino group, a dicyclohexylamino group, a diphenylamino group, a distyrenylamino group, a dinaphthylamino group, a dibenzylamino group, a dianthracenylamino group, a methylphenylamino group, an ethylbutylamino group, a phenylcyclohexylamino group, a t-butylnaphthylamino group, and a benzylanthracenylamino group.
Examples of the acyl group include an acetyl group, a propanoyl group, a butanoyl group, an octanoyl group, a benzoyl group, toluoyl group, a xyloyl group, a naphthoyl group, a phenanthroyl group, an anthroyl group and the like.

Of these, the ring A is preferably a pyrrolidine skeleton which may have a substituent group and imidazolidinone skeletons which may have a substituent group.

Next, as the groups represented by R¹ and R², the alkyl group, cycloalkyl group, alkenyl group, aryl group, aralkyl group, alkoxy group, carboxyl group, alkoxycarbonyl group, amide group, carbamoyl group and siloxy group will be described. These groups may have a substituent group.

As the alkyl group, examples thereof include a straight chain or branched chain alkyl group having, for example, from 1 to 30 carbon atoms, preferably from 1 to 10 carbon atoms, and illustrative examples thereof include methyl group, ethyl group, n-propyl group, isopropyl group, n-butyl group, 2-butyl group, isobutyl group, tert-butyl group, n-pentyl group, 2-pentyl group, 3-pentyl group, tert-pentyl group, 2-methylbutyl group, 3-methylbutyl group, 2,2-dimethylpropyl group, 1,2-dimethylpropyl group, n-hexyl group, 2-hexyl group, 3-hexyl group, 2-methylpentyl group, 3-methylpentyl group, 4-methylpentyl group, 1,1-dimethylbutyl group, 1,2-dimethylbutyl group, 1,3-dimethylbutyl group, 2,2-dimethylbutyl group, 2,3-dimethylbutyl group, 3,3-dimethylbutyl group, 2-ethylbutyl group, 1,1,2-trimethylpropyl group, 1,2,2-trimethylpropyl group, 1-ethyl-1-methylpropyl group, 1-ethyl-2-methylpropyl group, heptyl group, octyl group, nonyl group, decyl group, undecyl group, dodecyl group, tridecyl group, tetradecyl group, pentadecyl group, hexadecyl group, heptadecyl group, octadecyl group, nonadecyl group, icosyl group, henicosyl group, docosyl group and the like.

In addition, these alkyl groups may have a substituent group, and as said substituent group of alkyl groups, examples thereof include an alkenyl group, an alkynyl group, an aryl group, an aliphatic heterocyclic group, an aromatic heterocyclic group, a hydroxy group, an alkoxy group, a trialkylsiloxy group, an alkylenedioxy group, an aryloxy group, an aralkyloxy group, a heteroaryloxy group, a substituted amino group, a perhalogenoalkyl group, a halogen atom and the like.

As the alkenyl group as a substituent group of alkyl group, examples thereof include a straight chain or branched chain alkenyl group having, for example, from 2 to 20 carbon atoms, preferably from 2 to 10 carbon atoms, more preferably from 2 to 6 carbon atoms, and illustrative examples thereof include vinyl group, propenyl group, 1-butenyl group, pentenyl group, hexenyl group and the like.

As the alkynyl group as a substituent group of alkyl group, examples thereof include a straight chain or branched chain alkynyl group having, for example, from 2 to 15 carbon atoms, preferably from 2 to 10 carbon atoms, more preferably from 2 to 6 carbon atoms, and illustrative examples thereof include ethynyl group, 1-propynyl group, 2-propynyl group, 1-butynyl group, 3-butynyl group, pentinyl group, hexynyl group and the like.

As the aryl group as a substutient group of alkyl group, examples thereof include an aryl group having, for example, 6 to 14 carbon atoms, and illustrative examples thereof include a phenyl group, a naphthyl group, an anthryl group, a phenanthryl group, a biphenyl group, a tolyl group, a xylyl group, a mesityl group, a methoxyphenyl group, a dimethoxyphenyl group, a fluorophenyl group and the like.

As the aliphatic heterocyclic group as a substituent group of alkyl group, examples thereof include a group which has, for example, from 2 to 14 carbon atoms and contains, as heterogeneous atoms, at least one, preferably from 1 to 3 hetero atoms such as nitrogen atom, oxygen atom and sulfur atom. Preferably, a 5- or 6-membered monocyclic aliphatic heterocyclic group and a polycyclic or condensed ring aliphatic heterocyclic group can be mentioned. Illustrative examples of the aliphatic heterocyclic group include 2-oxo-1-pyrrolidinyl group, piperidino group, piperazinyl group, morpholino group, tetrahydrofuryl group, tetrahydropyranyl group, tetrahydrothienyl group and the like.

As the aromatic heterocyclic group as a substituent group of alkyl group, examples thereof include a group which has, for example, from 2 to 15 carbon atoms and contains, as heterogeneous atoms, at least one, preferably from 1 to 3 hetero atoms such as nitrogen atom, oxygen atom and sulfur atom. Preferably, a 5- or 6-membered monocyclic aromatic heterocyclic group and a polycyclic or condensed ring aromatic heterocyclic group can be mentioned. Illustrative examples of the aromatic heterocyclic group include furyl group, thienyl group, pyridyl group, pyrimidinyl group, pyrazinyl group, pyridazinyl group, pyrazolinyl group, imidazolyl group, oxazolinyl group, thiazolinyl group, benzofuryl group, benzothienyl group, quinolyl group, isoquinolyl group, quinoxalinyl group, phtharazinyl group, quinazolinyl group, naphthylidinyl group, cinnolinyl group, benzimidazolyl group, benzoxazolyl group, benzothiazolyl group and the like.

As the alkoxy group as a substituent group of alkyl group, examples thereof include a straight chain or branched chain alkoxy group having, for example, from 1 to 8 carbon atoms, and illustrative examples thereof include methoxy group, ethoxy group, n-propoxy group, isopropoxy group, n-butoxy group, 2-butoxy group, isobutoxy group, tert-butoxy group, n-pentyloxy group, 2-methylbutoxy group, 3-methylbutoxy group, 2,2-dimethylpropoxy group, n-hexyloxy group, 2-methylpentyloxy group, 3-methylpentyloxy group, 4-methylpentyloxy group, 5-methylpentyloxy group, cyclopentyloxy group, cyclohexyloxy group and the like.

As the trialkylsiloxy group as a substituent group of alkyl group, examples thereof include trimethylsiloxy group, triethylsiloxy group, dimethyl-tert-butylsiloxy group and the like.

As the alkylenedioxy group as a substituent group of alkyl group, examples thereof include an alkylenedioxy group having, for example, from 1 to 3 carbon atoms, and illustrative examples thereof include methylenedioxy group, ethylenedioxy group, propylenedioxy group, isopropylidenedioxy group and the like.

As the aryloxy group as a substituent group of alkyl group, examples thereof include an aryloxy group having, for example, from 6 to 15 carbon atoms can be mentioned, and illustratively, phenoxy group, naphthyloxy group, anthryloxy group, tolyloxy group, xylyloxy group, 4-phenylphenoxy group, 3,5-diphenylphenoxy group, 4-mesitylphenoxy group, 3,5-bis(trifluoromethyl)phenoxy group and the like.

As the aralkyloxy group as a substituent group of alkyl group, examples thereof include an aralkyloxy group having from 7 to 12 carbon atom, and illustrative examples thereof include benzyloxy group, 2-phenylethoxy group, 1-phenylpropoxy group, 2-phenylpropoxy group, 3-phenylpropoxy group, 1-phenylbutoxy group, 2-phenylbutoxy group, 3-phenylbutoxy group, 4-phenylbutoxy group, 1-phenylpentyloxy group, 2-phenylpentyloxy group, 3-phenylpentyloxy group, 4-phenylpentyloxy group, 5-phenylpentyloxy group, 1-phenylhexyloxy group, 2-phenylhexyloxy group, 3-phenylhexyloxy group, 4-phenylhexyloxy group, 5-phenylhexyloxy group, 6-phenylhexyloxy group and the like.

As the heteroaryloxy group as a substituent group of alkyl group, examples thereof include a heteroaryloxy group which has, for example, from 2 to 14 carbon atoms and contains, as heterogeneous atoms, at least one, preferably from 1 to 3 hetero atoms such as nitrogen atom, oxygen atom and sulfur atom, and illustrative examples thereof include 2-pyridyloxy group, 2-pyrazyloxy group, 2-pyrimidyloxy group, 2-quinolyloxy group and the like.

As the substituted amino group as a substituent group of alkyl group, examples thereof include mono- or di-alkylamino groups such as N-methylamino group, N,N-dimethylamino group, N,N-diethylamino group, N,N-diisopropylamino group, N-cyclohexylamino group, pyrrolidyl group, piperidyl group and morpholyl group; mono- or di-arylamino group such as N-phenylamino group, N,N-diphenylamino group, N-naphthylamino group and N-naphthyl-N-phenylamino group; mono- or di-aralkylamino group such as N-benzylamino group and N,N-dibenzylamino group; and the like.

As the perhalogenoalkyl group substituting an alkyl group, examples thereof include a trifluoromethyl group, a pentafluoroethyl group, a heptafluoropropyl group, a nonafluorobutyl group, a trichloromethyl group, a pentachloroethyl group and the like.

As the halogen atom substituting the alkyl group, examples thereof include fluorine atom, chlorine atom, bromine atom, iodine atom and the like.

As the cycloalkyl group, examples thereof include cyclopropyl group, cyclobutyl group, cyclopentyl group, cyclohexyl group and the like.
These cycloalkyl groups may have a substituent group, and as said substituent group, the substituent groups described in the aforementioned description on the substituent group of alkyl group can be mentioned.

As the alkenyl group, examples thereof include a straight or branched chain or cyclic alkenyl group having, for example, from 2 to 20 carbon atoms, preferably from 2 to 10 carbon atoms. Illustrative examples of alkenyl groups include vinyl group, 1-propenyl group, 2-propenyl group, 1-butenyl group, 2-butenyl group, 3-butenyl group, 1-pentenyl group, 2-pentenyl group, 3-pentenyl group, 4-pentenyl group, 1-cyclopentenyl group, 3-cyclopentenyl group, 1-hexenyl group, 2-hexenyl group, 3-hexenyl group, 4-hexenyl group, 5-hexenyl group, 4-methyl-3-pentenyl group, 4,8-dimethyl-3,7-nonadienyl group, 1-cyclohexenyl group, 3-cyclohexenyl group and the like.
These alkenyl groups may have a substituent group, and as said substituent group, the groups described in the aforementioned description on the substituent group of alkyl group can be mentioned.

As the aryl group, examples thereof include an aryl group having 6 to 14 carbon atoms, and specific examples thereof include a phenyl group, a naphthyl group, an anthryl group, a phenanthryl group, a biphenyl group and the like. These aryl groups may have a substituent group and examples of the substituent group are the same as those mentioned in the description of substituent group of the alkyl group.

As the aralkyl group, an aralkyl group having, for example, from 7 to 45 carbon atoms is desirable, and illustrative examples thereof include benzyl group, tolylmethyl group, xylylmethyl group, mesitylmethyl group, 4-phenylphenylmethyl group, 3-phenylphenylmethyl group, 2-phenylphenylmethyl group, 4-mesitylphenylmethyl group, 1-naphthylmethyl group, 2-naphthylmethyl group, 9-anthranylmethyl group, 9-phenanthrylmethyl group, 3,5-diphenylphenylmethyl group, 2-phenylethyl group, 1-phenylpropyl group, 3-naphthylpropyl group, diphenylmethyl group, ditolylmethyl group, dixylylmethyl group, dimesitylmethyl group, di(4-phenylphenyl)methyl group, di(3-phenylphenyl)methyl group, di(2-phenylphenyl)methyl group, di(4-mesitylphenyl)methyl group, di-1-naphthylmethyl group, di-2-naphthylmethyl group, di-9-anthranylmethyl group, di-9-phenanthrylmethyl group, bis(3,5-diphenylphenyl)methyl group, triphenylmethyl group, tritolylmethyl group, trixylylmethyl group, trimesitylmethyl group, tri(4-phenylphenyl)methyl group, tri(3-phenylphenyl)methyl group, tri(2-phenylphenyl)methyl group, tri(4-mesitylphenyl)methyl group, tri-1-naphthyl methyl group, tri-2-naphthylmethyl group, tri-9-anthranylmethyl group, tri-9-phenanthrylmethyl group, tris(3,5-diphenylphenyl)methyl group, hydroxyphenylmethyl group, hydroxydiphenylmethyl group, hydroxyditolylmethyl group, hydroxydi(4-t-butylphenyl)methyl group, hydroxydixylylmethyl group, hydroxydi(2-phenylphenyl)methyl group, hydroxydi(3-phenylphenyl)methyl group, hydroxydi(4-phenylphenyl)methyl group, hydroxybis(3,5-diphenylphenyl)methyl group, hydroxydi(4-mesitylphenyl)methyl group, hydroxybis(3,5-ditrifluoromethylphenyl)methyl group, trimethylsiloxyphenylmethyl group, trimethylsiloxydiphenyl methyl group, trimethylsiloxyditolyl methyl group, trimethyl siloxydi(4-t-butylphenyl)methyl group, trimethylsiloxydixylylmethyl group, trimethylsiloxydi(2-phenylphenyl)methyl group, trimethylsiloxydi(3-phenylphenyl)methyl group, trimethylsiloxydi(4-phenylphenyl)methyl group, trimethylsiloxybis(3,5-diphenylphenyl)methyl group, trimethylsiloxydi(4-mesitylphenyl)methyl group, trimethylsiloxybis(3,5-ditrifluoromethylphenyl)methyl group and the like.
These aralkyl groups may have a substituent group, and as said substituent group, the groups described in the description on the substituent group of alkyl group can be mentioned.

As the alkoxy group, an alkoxy group having, for example, from 1 to 30 carbon atoms is desirable, and illustrative examples thereof include methoxy group, ethoxy group, n-propoxy group, isopropoxy group, n-butoxy group, 2-butoxy group, isobutoxy group, tert-butoxy group, n-pentyloxy group, 2-methylbutoxy group, 3-methylbutoxy group, 2,2-dimethylpropoxy group, n-hexyloxy group, 2-methylpentyloxy group, 3-methylpentyloxy group, 4-methylpentyloxy group, 5-methylpentyloxy group, cyclopentyloxy group, cyclohexyloxy group, dicyclopentylmethoxy group, dicyclohexylmethoxy group, tricyclopentyl methoxy group, tricyclohexylmethoxy group, phenylmethoxy group, diphenylmethoxy group, triphenylmethoxy group and the like.
These alkoxy groups may have a substituent group, and as said substituent group, the groups described in the description on the substituent group of alkyl group can be mentioned.

As the carboxyl group, a carboxyl group having, for example, from 1 to 30 carbon atoms is desirable, and illustrative examples thereof include acetoxy group, n-propanoyloxy group, isopropanoyloxy group, n-butanoyloxy group, 2-butanoyloxy group, isobutanoyloxy group, tert-butanoyloxy group, n-pentanoyloxy group, 2-methylbutanoyloxy group, 3-methylbutanoyloxy group, 2,2-dimethylpropanoyloxy group, n-hexanoyloxy group, 2-methylpentanoyloxy group, 3-methylpentanoyloxy group, 4-methylpentanoyloxy group, 5-methylpentanoyloxy group, cyclopentanoyloxy group, cyclohexanoyloxy group, dicyclopentylacetoxy group, dicyclohexylacetoxy group, tricyclopentylacetoxy group, tricyclohexylacetoxy group, phenylacetoxy group, diphenylacetoxy group, triphenylacetoxy group, benzoyloxy group, naphthoyloxy group and the like.
These carboxyl groups may have a substituent group, and as said substituent group, the groups described in the description on the substituent group of alkyl group can be mentioned.

As the alkoxycarbonyl group, an alkoxycarbonyl group having, for example, from 1 to 30 carbon atoms is desirable, and illustrative examples thereof include methoxycarbonyl group, ethoxycarbonyl group, n-propoxycarbonyl group, isopropoxycarbonyl group, n-butoxycarbonyl group, 2-butoxycarbonyl group, isobutoxycarbonyl group, tert-butoxycarbonyl group, n-pentyloxycarbonyl group, 2-methylbutoxycarbonyl group, 3-methylbutoxycarbonyl group, 2,2-dimethylpropoxycarbonyl group, n-hexyloxycarbonyl group, 2-methylpentyloxycarbonyl group, 3-methylpentyloxycarbonyl group, 4-methylpentyloxycarbonyl group, 5-methylpentyloxycarbonyl group, cyclopentyloxycarbonyl group, cyclohexyloxycarbonyl group, dicyclopentylmethoxycarbonyl group, dicyclohexylmethoxycarbonyl group, tricyclopentylmethoxycarbonyl group, tricyclohexylmethoxycarbonyl group, phenylmethoxycarbonyl group, diphenylmethoxycarbonyl group, triphenylmethoxycarbonyl group and the like.
These alkoxycarbonyl groups may have a substituent group, and as said substituent group, the groups described in the description on the substituent group of alkyl group can be mentioned.

As the amido group, an amido group having, for example, from 1 to 30 carbon atoms is desirable, and illustrative examples thereof include acetamido group, n-propionamido group, isopropionamido group, n-butanamido group, 2-butanamido group, isobutanamido group, tert-butanamido group, n-pentanamido group, 2-methylbutanamido group, 3-methylbutanamido group, 2,2-dimethyl propionamido group, n-hexanamido group, 2-methylpentanamido group, 3-methylpentanamido group, 4-methylpentanamido group, 5-methylpentanamido group, cyclopentanamido group, cyclohexanamido group, dicyclopentylacetamido group, dicyclohexylacetamido group, tricyclopentylacetamido group, tricyclohexylacetamido group, phenylacetamido group, diphenylacetamido group, triphenylacetamido group, benzamido group, naphthalenamido group and the like.
These amido groups may have a substituent group, and as said substituent group, the groups described in the description on the substituent group of alkyl group can be mentioned.

As the carbamoyl group, examples thereof include a carbamoyl group having, for example, 1 to 30 carbon atoms, and specific examples thereof include carbamoyl group, methyl carbamoyl group, ethyl carbamoyl group, n-propyl carbamoyl group, isopropyl carbamoyl group, n-butyl carbamoyl group, 2-butyl carbamoyl group, isobutyl carbamoyl group, tert-butylcarbamoyl group, n-pentylcarbamoyl group, cyclopentylcarbamoyl group, cyclohexylcarbamoyl group, cycloheptylcarbamoyl group, dimethylcarbamoyl group, diethylcarbamoyl group, di-n-propylcarbamoyl group, diisopropylcarbamoyl group, di-n-butylcarbamoyl group, di-2-butylcarbamoyl group, diisobutylcarbamoyl group, di-tert-butylcarbamoyl group, di-n-pentylcarbamoyl group, dicyclopentylcarbamoyl group, dicyclohexylcarbamoyl group, dicycloheptylcarbamoyl group, N,N-1,4-tetramethylenecarbamoyl group, N,N-1,5-pentamethylenecarbamoyl group, morpholinecarbamoyl group, phenylcarbamoyl group, tolylcarbamoyl group, a xylylcarbamoyl group, mesitylcarbamoyl group, 3,5-ditert-butylphenylcarbamoyl group, p-phenylcarbamoyl group, m-phenylcarbamoyl group, o-phenylcarbamoyl group, 4-phenylphenylcarbamoyl group, 3-phenylphenylcarbamoyl group, 2-phenylphenylcarbamoyl group, 4-mesitylphenylcarbamoyl group, 3,5-diphenylphenylcarbamoyl group, naphthylcarbamoyl group, phenanthrylcarbamoyl group, anthranylcarbamoyl group, diphenylcarbamoyl group, ditolylcarbamoyl group, dixylylcarbamoyl group, dimesitylcarbamoyl group, bis(3,5-ditert-butylphenyl)carbamoyl group, di-p-phenylcarbamoyl group, di-m-phenylcarbamoyl group, di-o-phenylcarbamoyl group, di(4-phenylphenyl)carbamoyl group, di(3-phenylphenyl)carbamoyl group, di(2-phenylphenyl)carbamoyl group, di(4-mesitylphenyl)carbamoyl group, bis(3,5-diphenylphenyl)carbamoyl group, dinaphthylcarbamoyl group, diphenanthrylcarbamoyl group and dianthranylcarbamoyl group and the like.
These carbamoyl groups may have a substituent group and examples of the substituent group are the same as those mentioned in the description of substituent group of the alkyl group.

As the siloxy group, examples thereof include trimethylsiloxy group, triethylsiloxy group, dimethyl-tert-butylsiloxy group and the like.
These siloxy groups may have a substituent group, and as said substituent group, the groups described in the description on the substituent group of alkyl group can be mentioned.

As illustrative optically active cyclic nitrogen-containing compounds, the following compounds can, for example, be mentioned.
In the following compounds, Me represents methyl group, Ph represents phenyl group, Bu represents butyl group, Bn represents benzyl group, Et represents ethyl group, TMS represents trimethylsilyl group, Pr represents propyl group, iPr represents isopropyl group, and polymer represents a polymer chain.

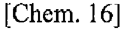

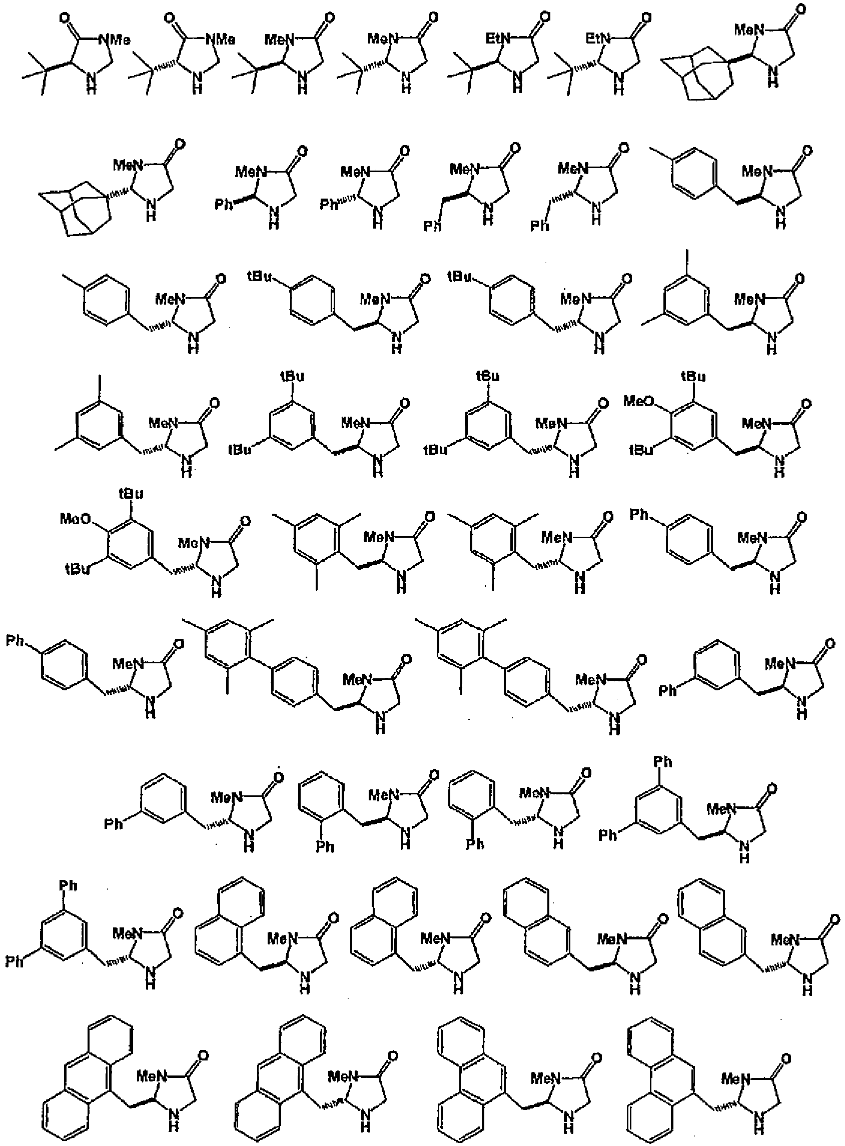

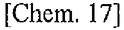

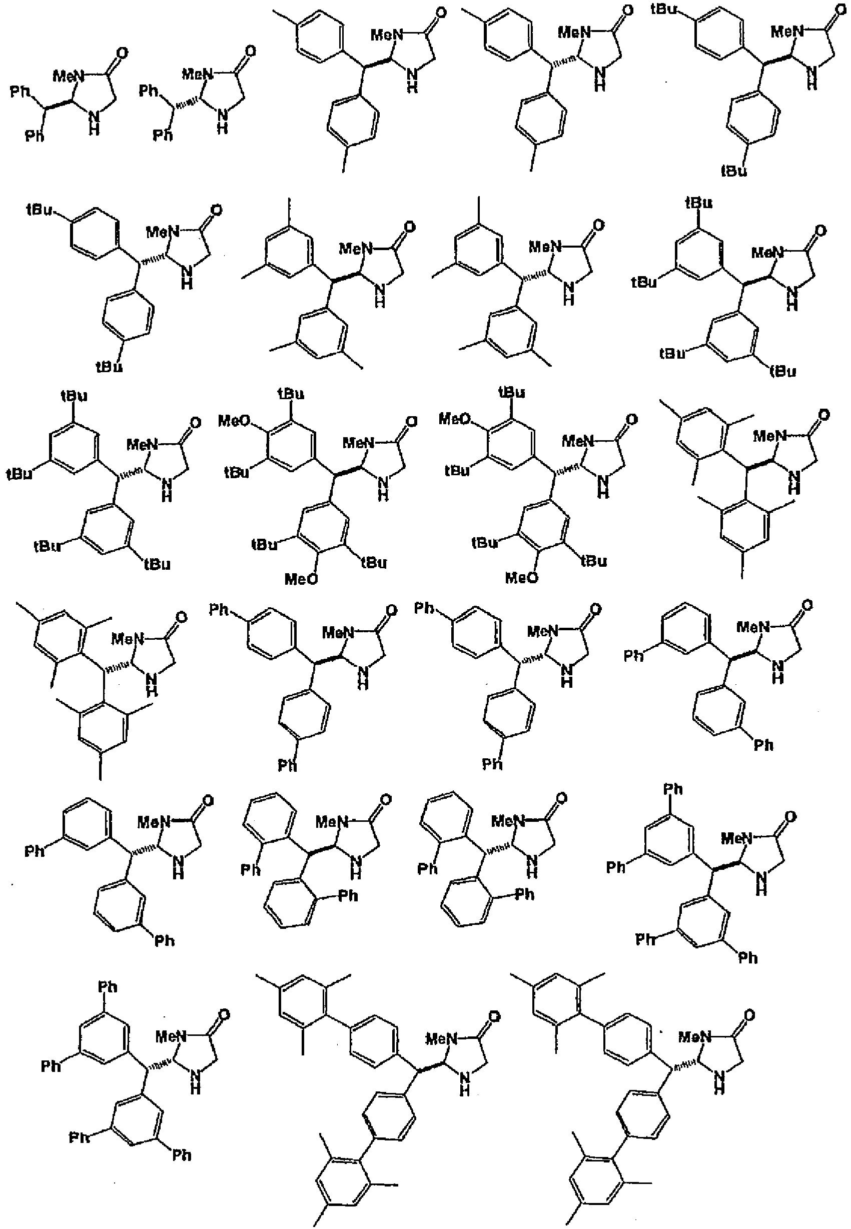

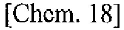

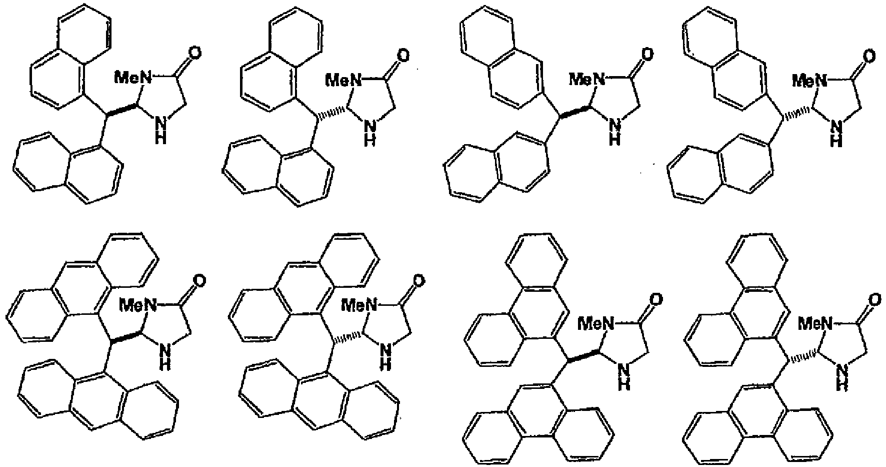

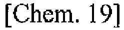

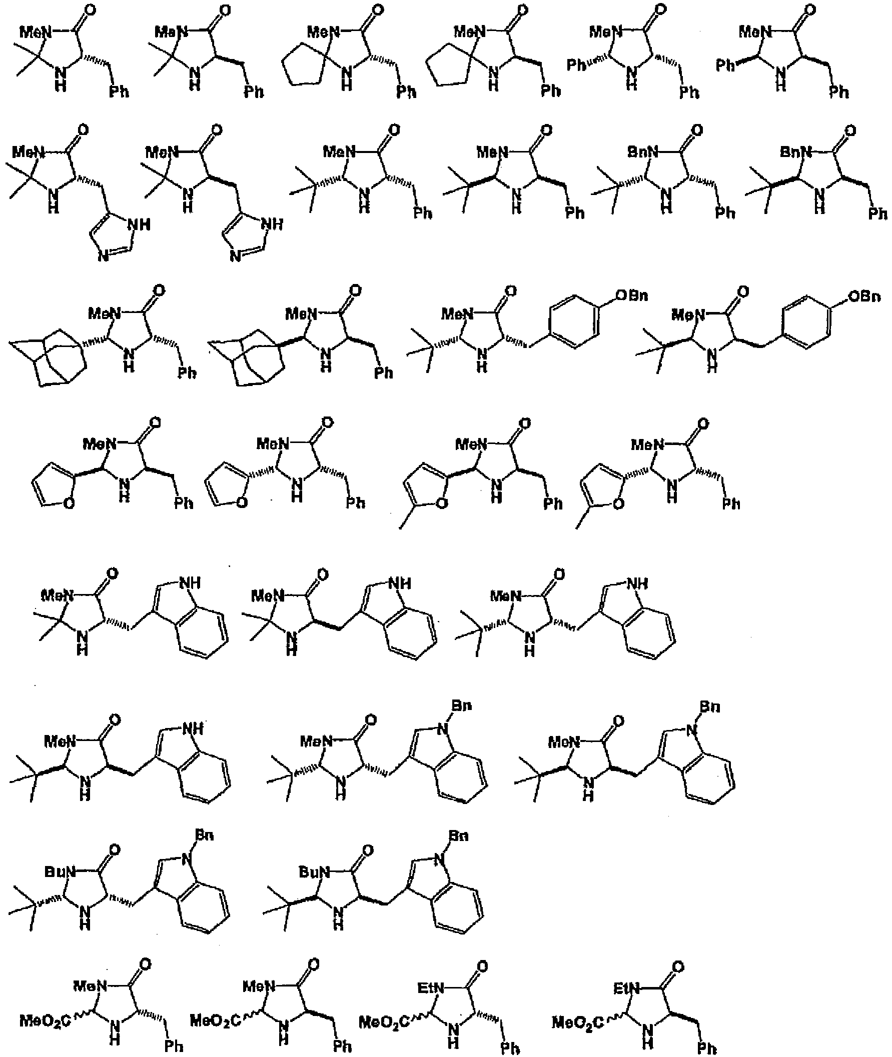

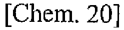

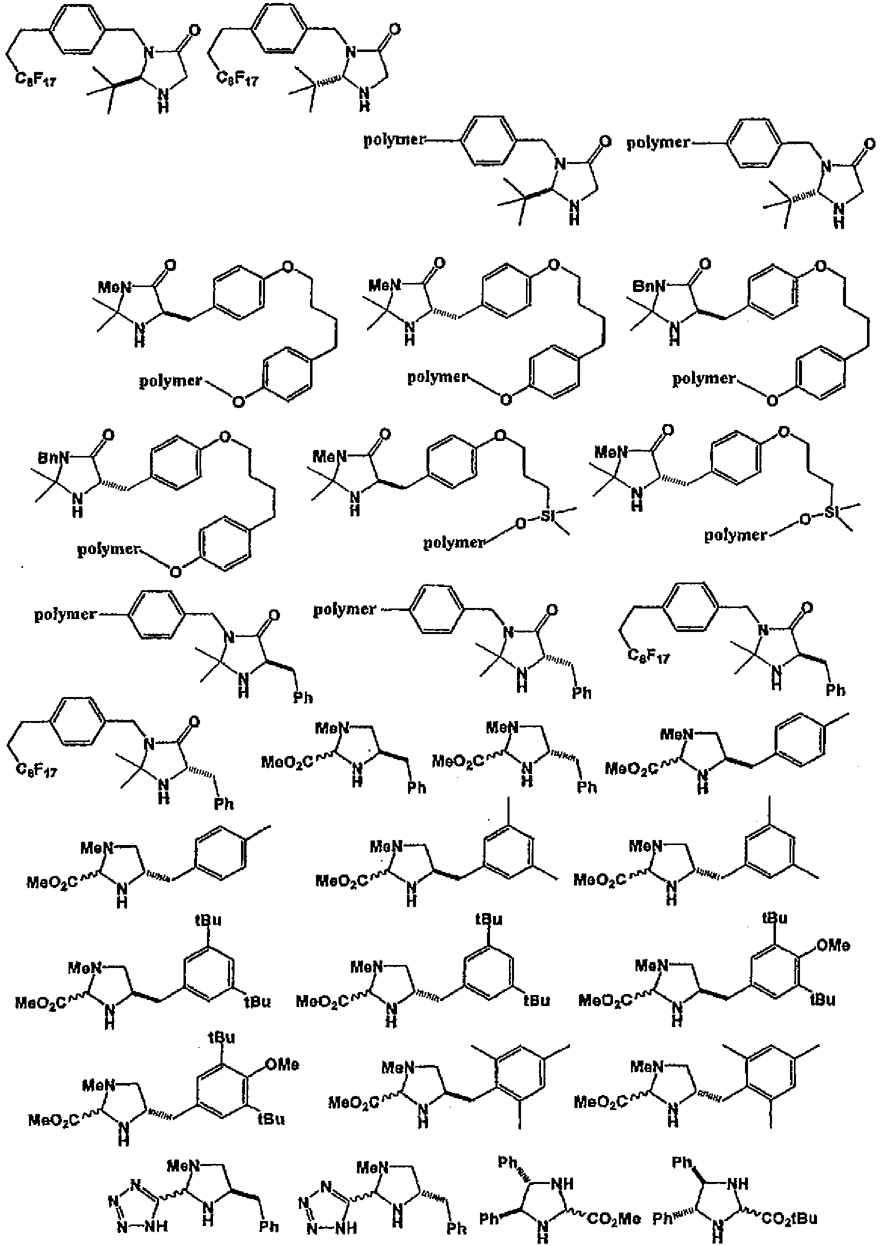

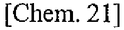

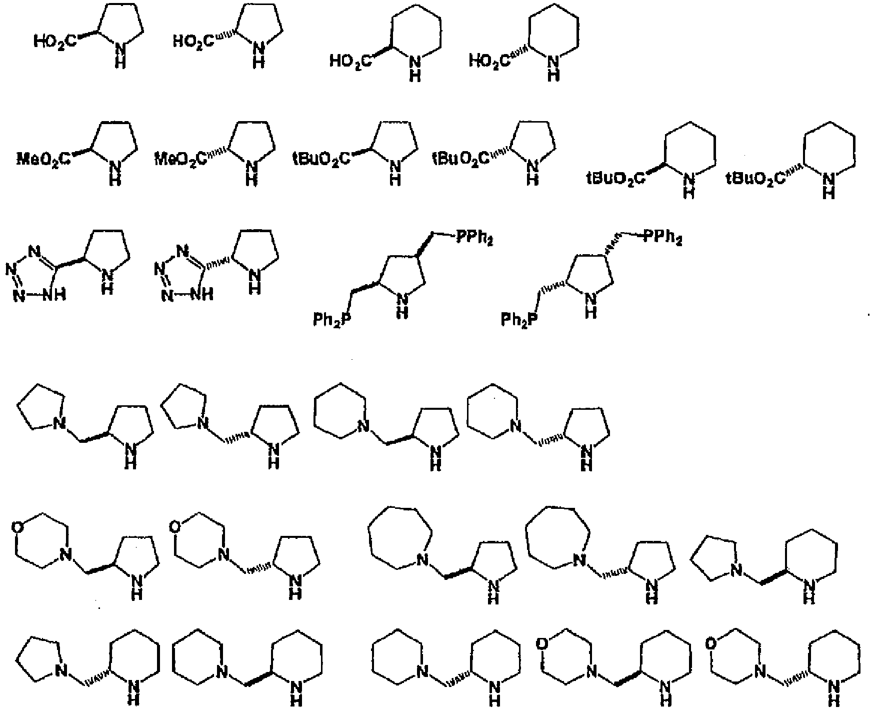

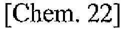

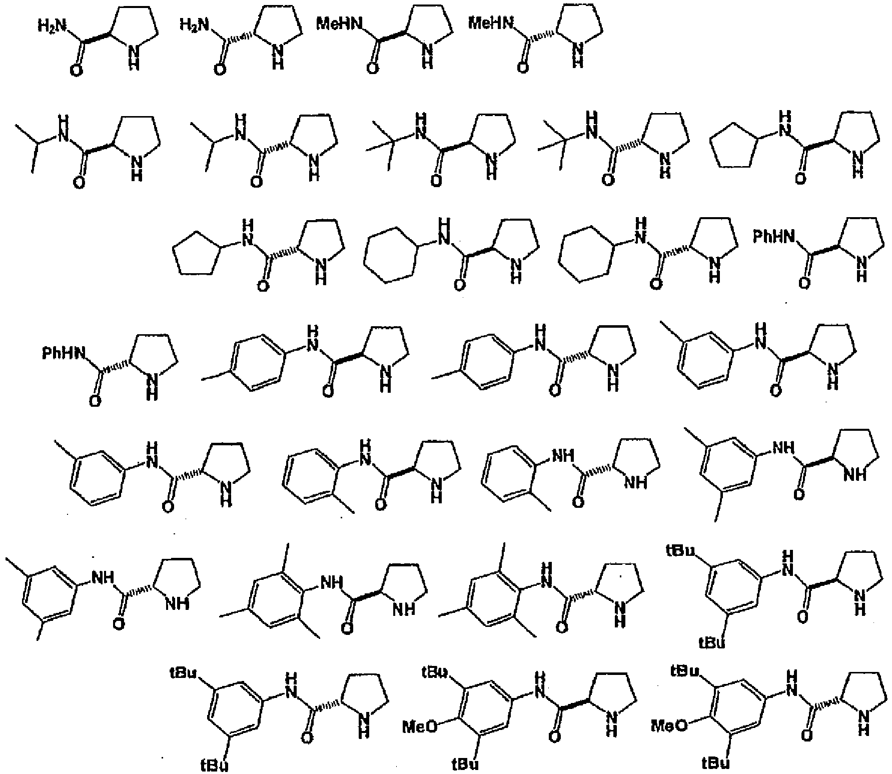

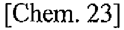

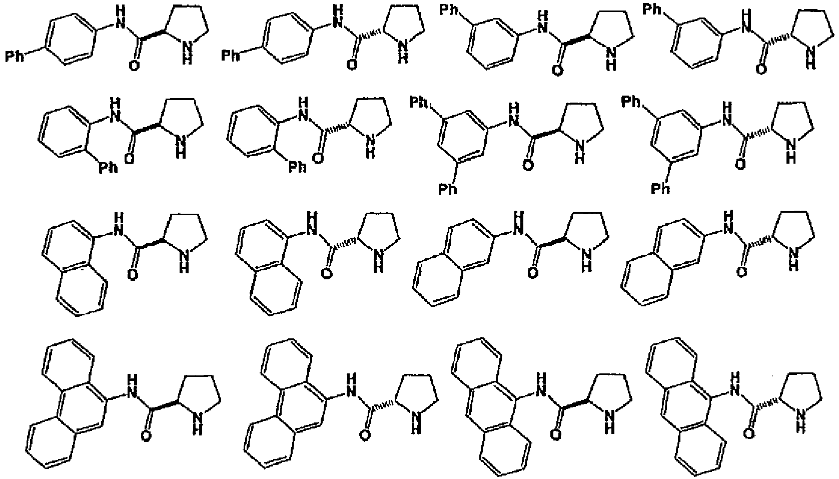

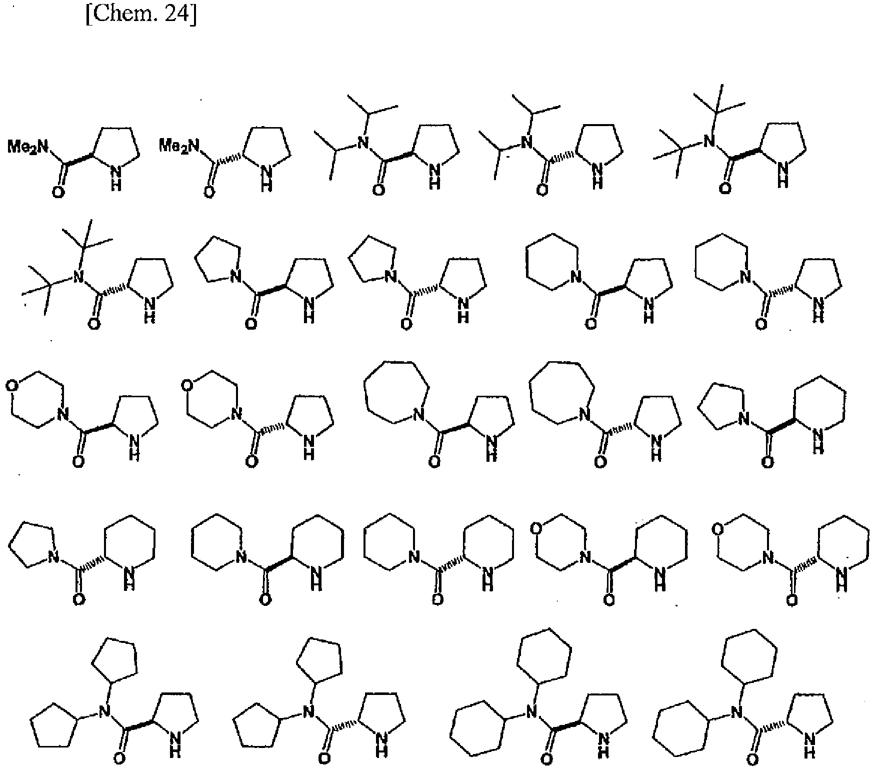

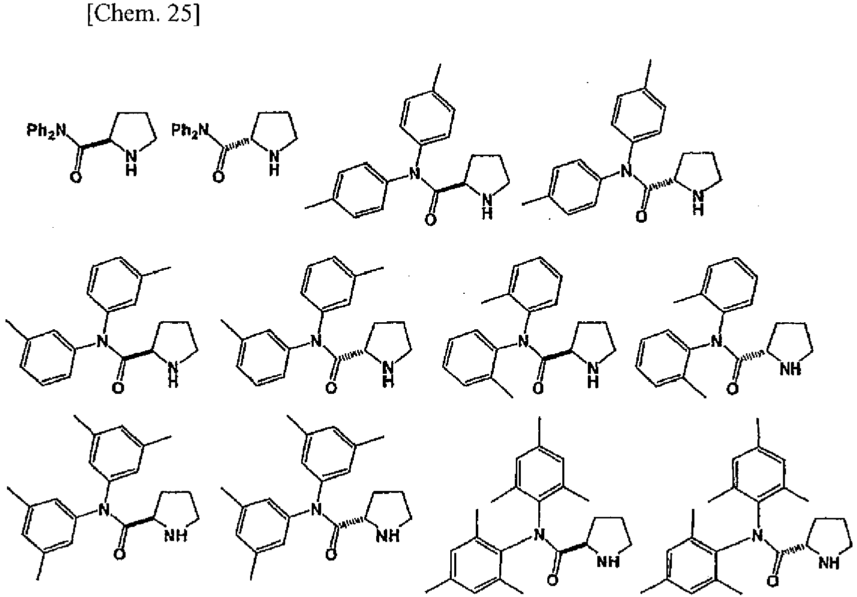

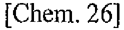

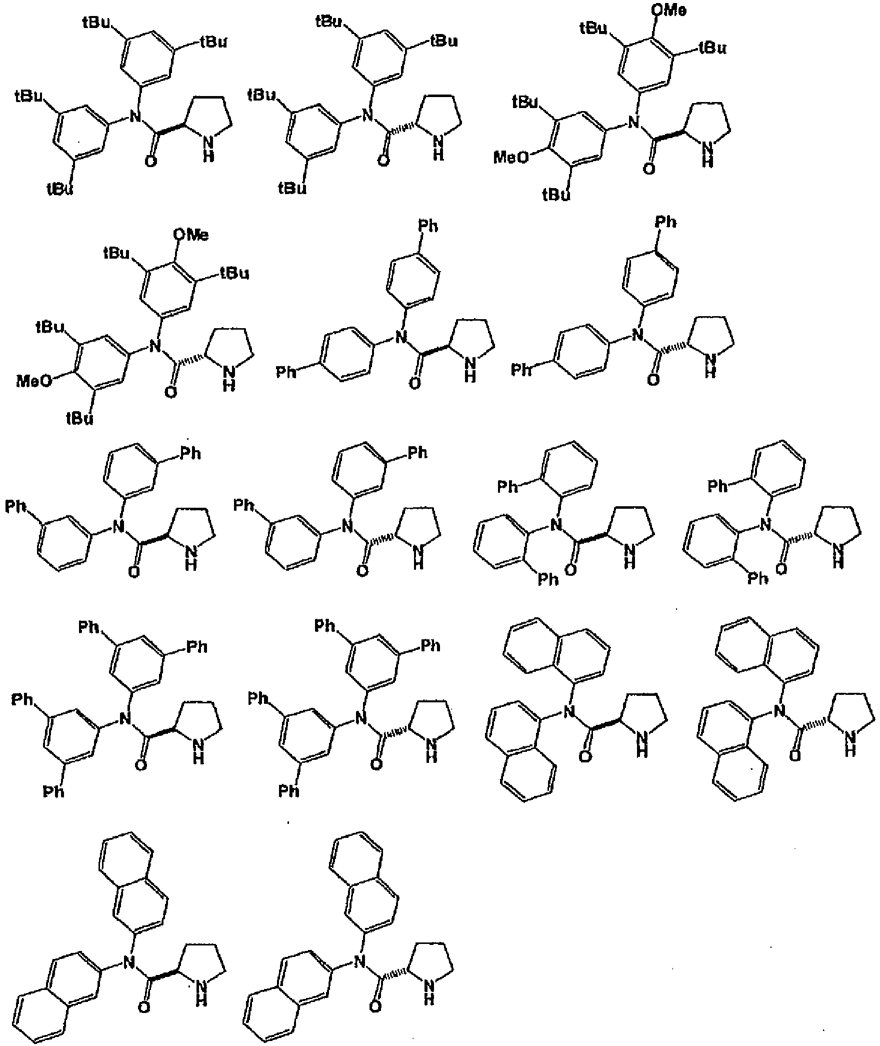

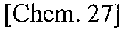

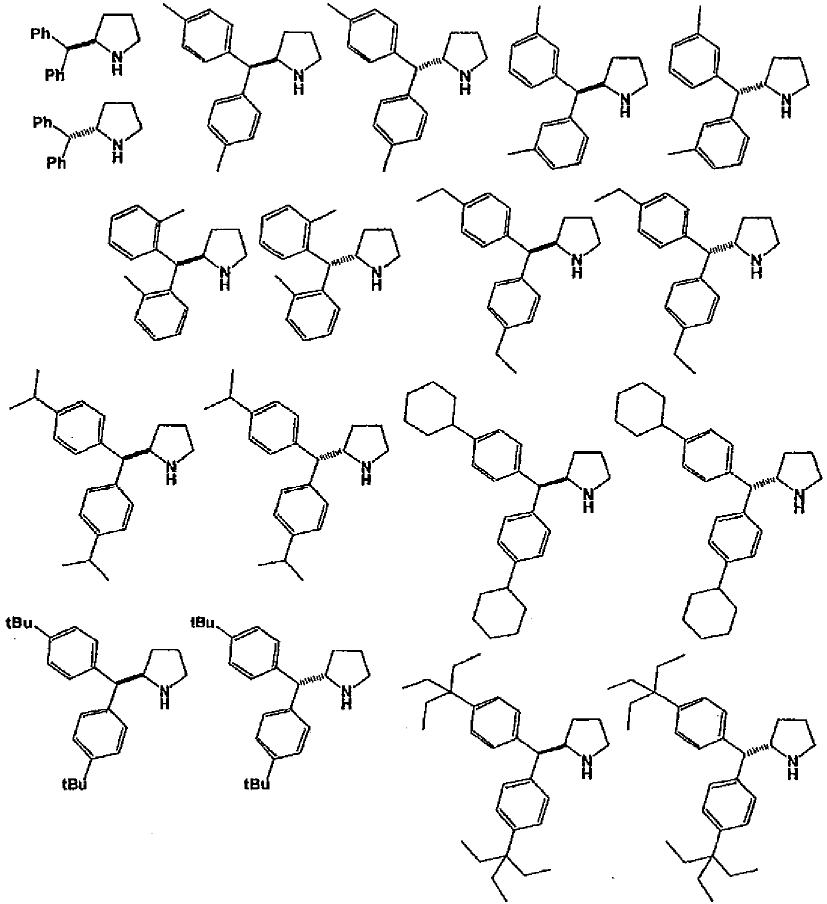

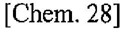

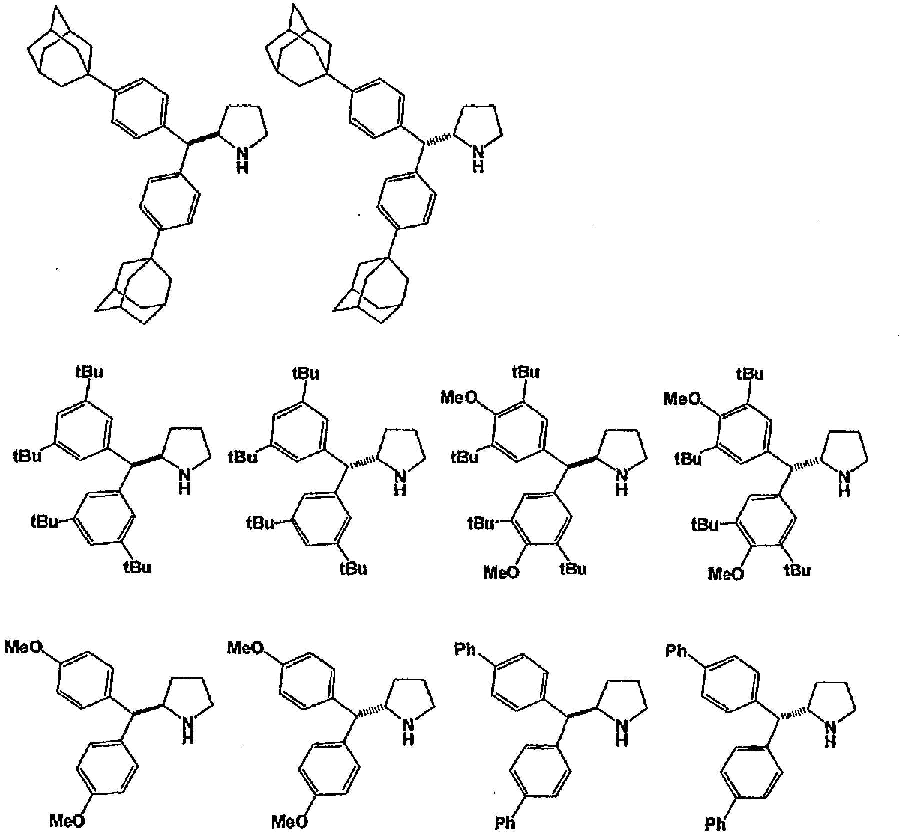

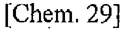

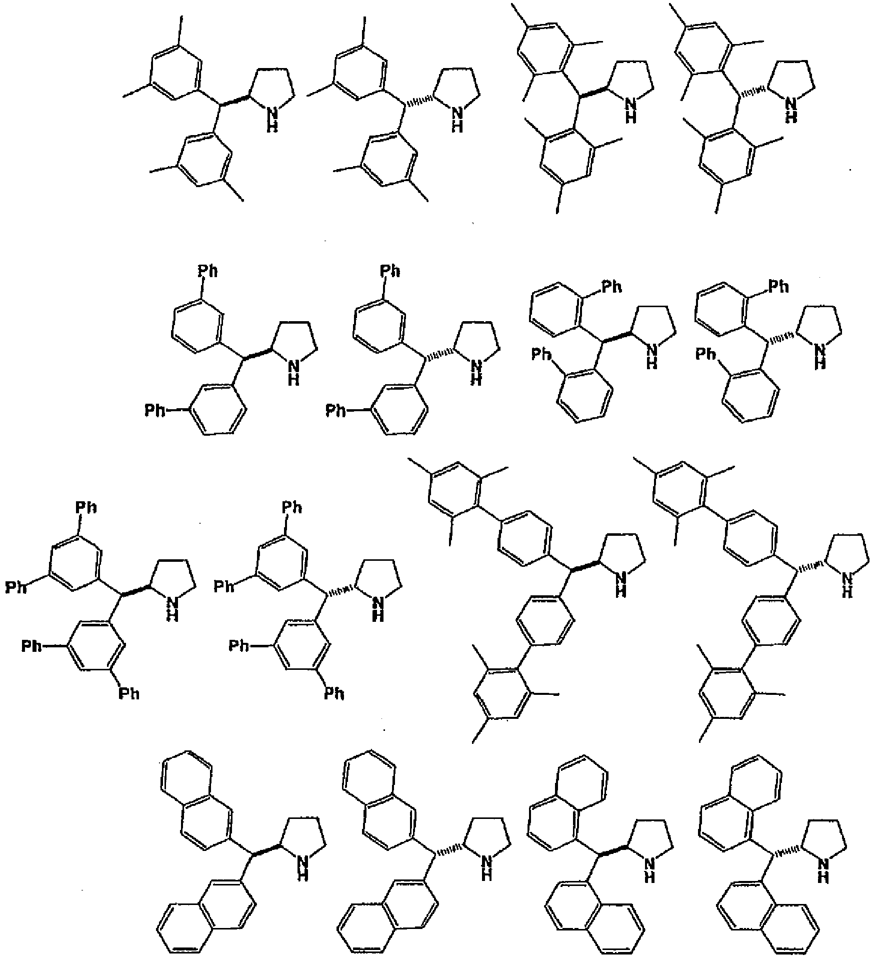

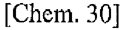

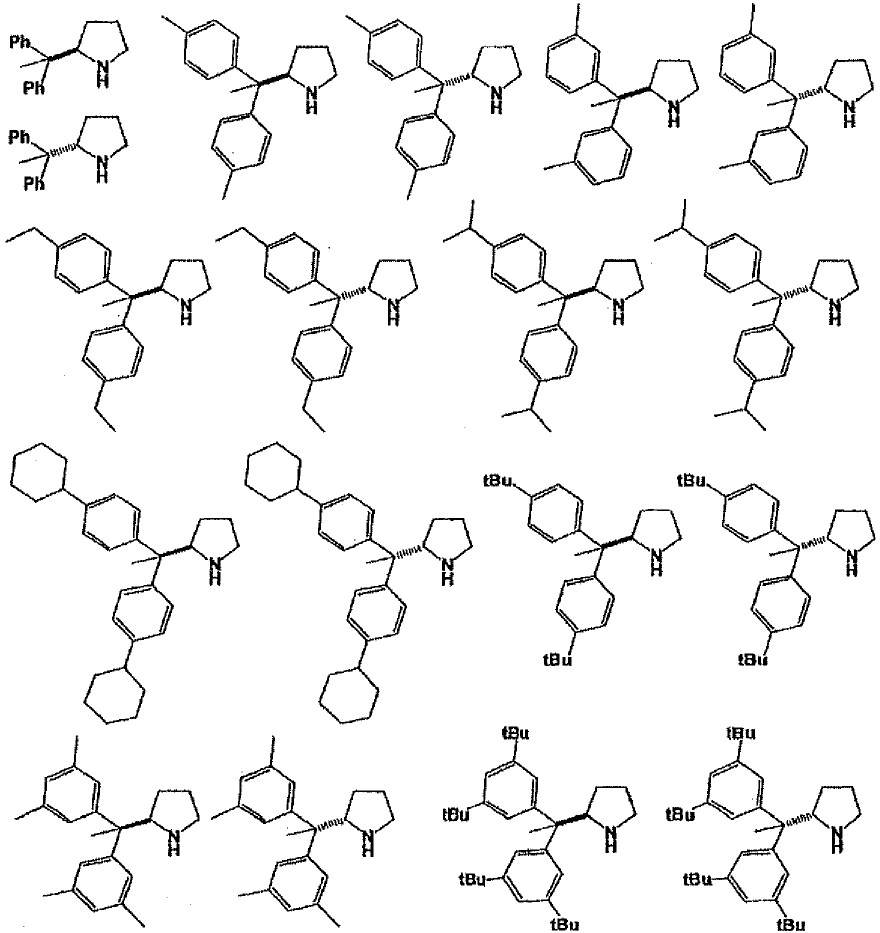

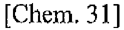

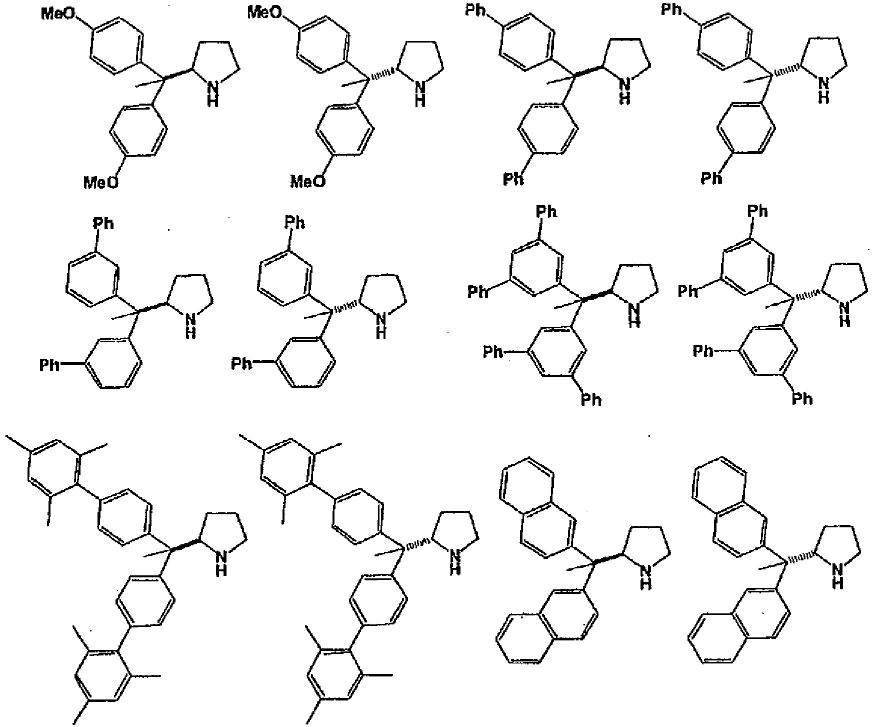

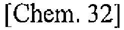

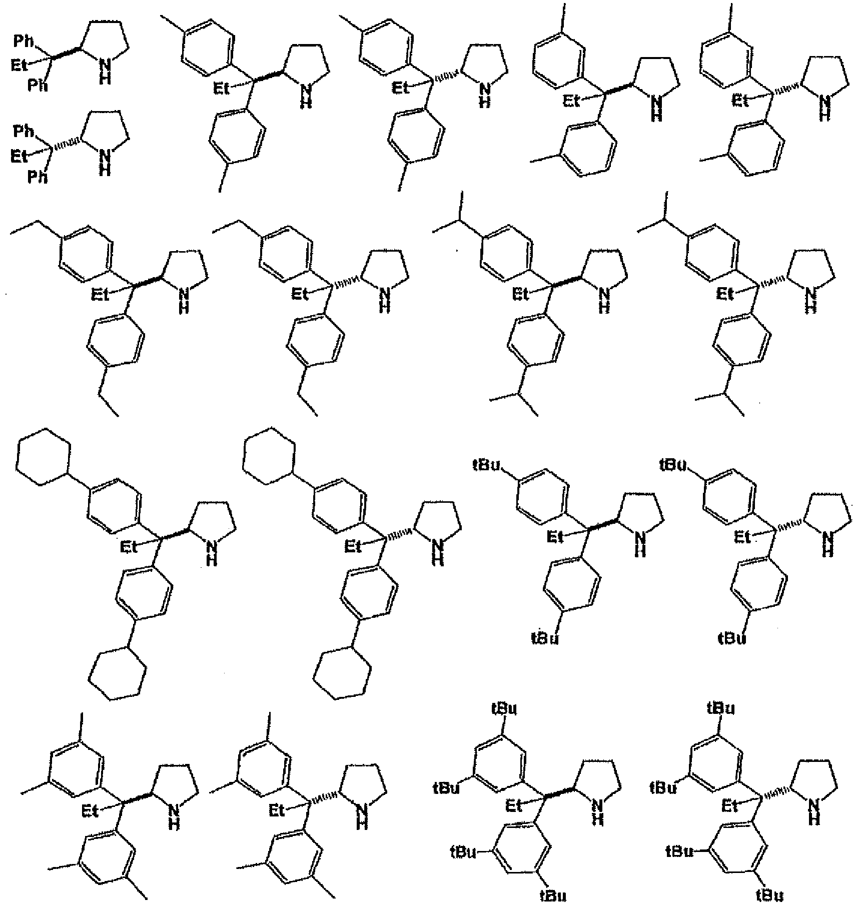

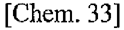

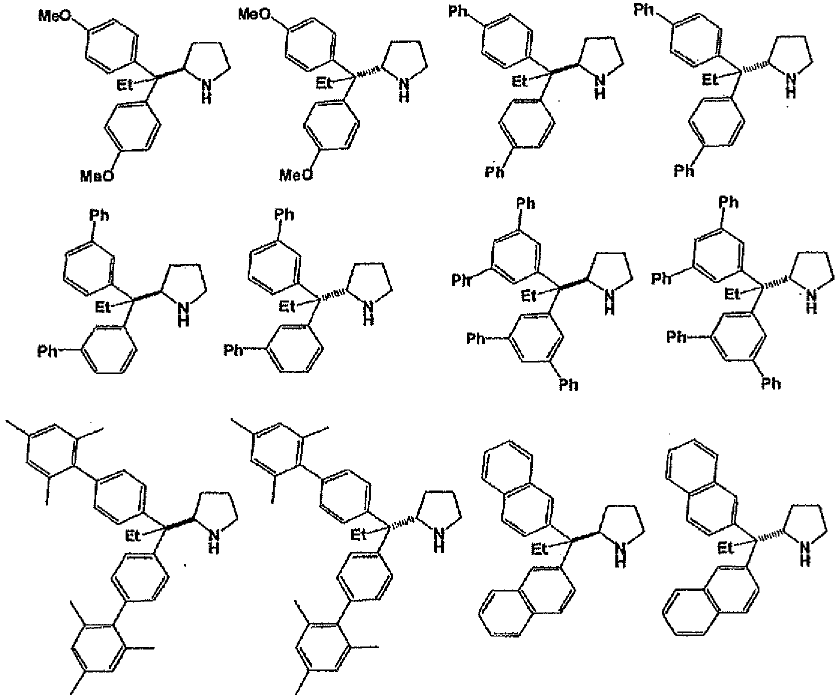

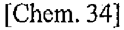

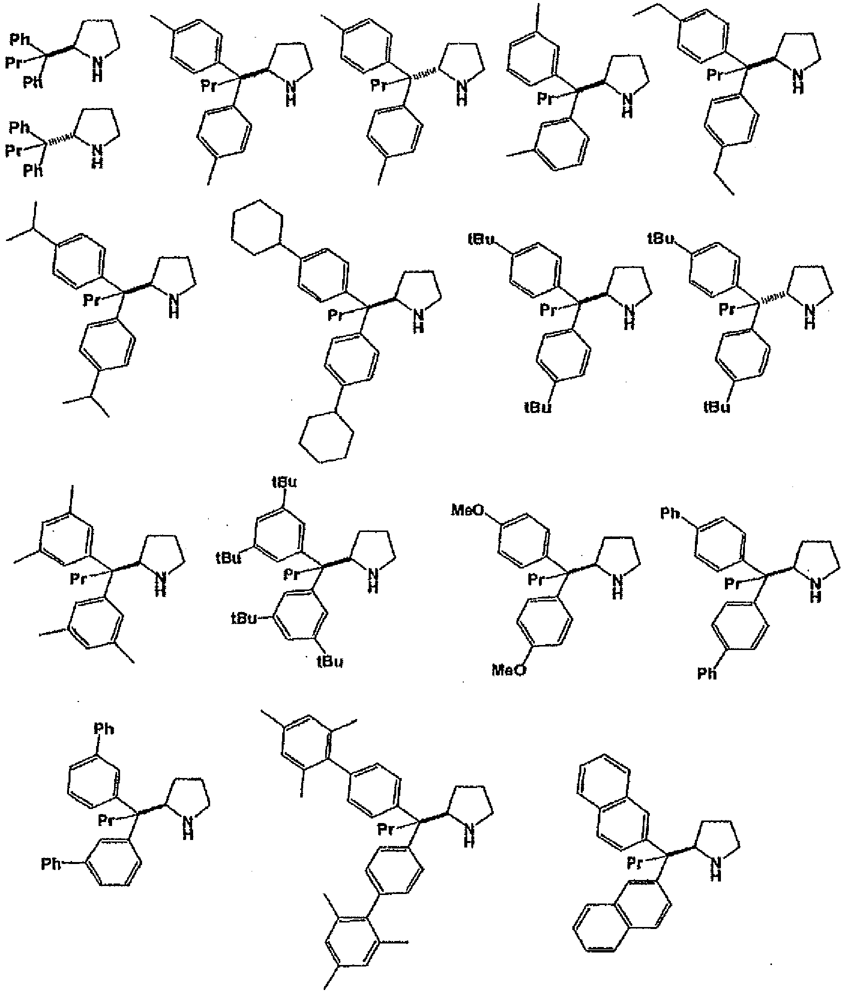

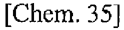

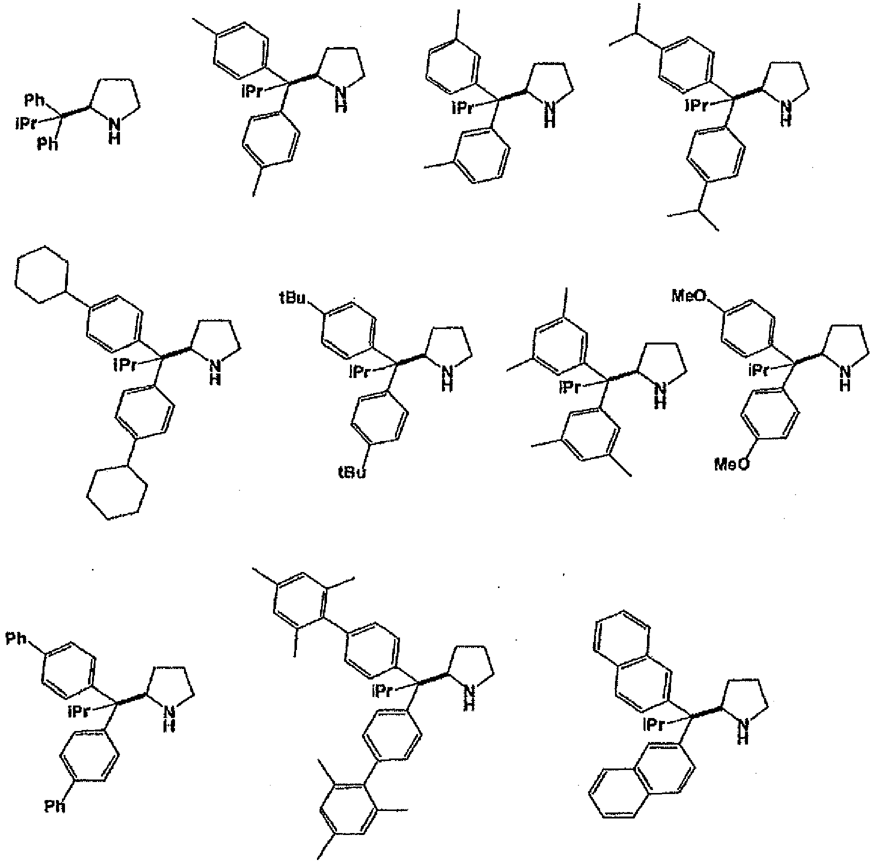

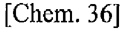

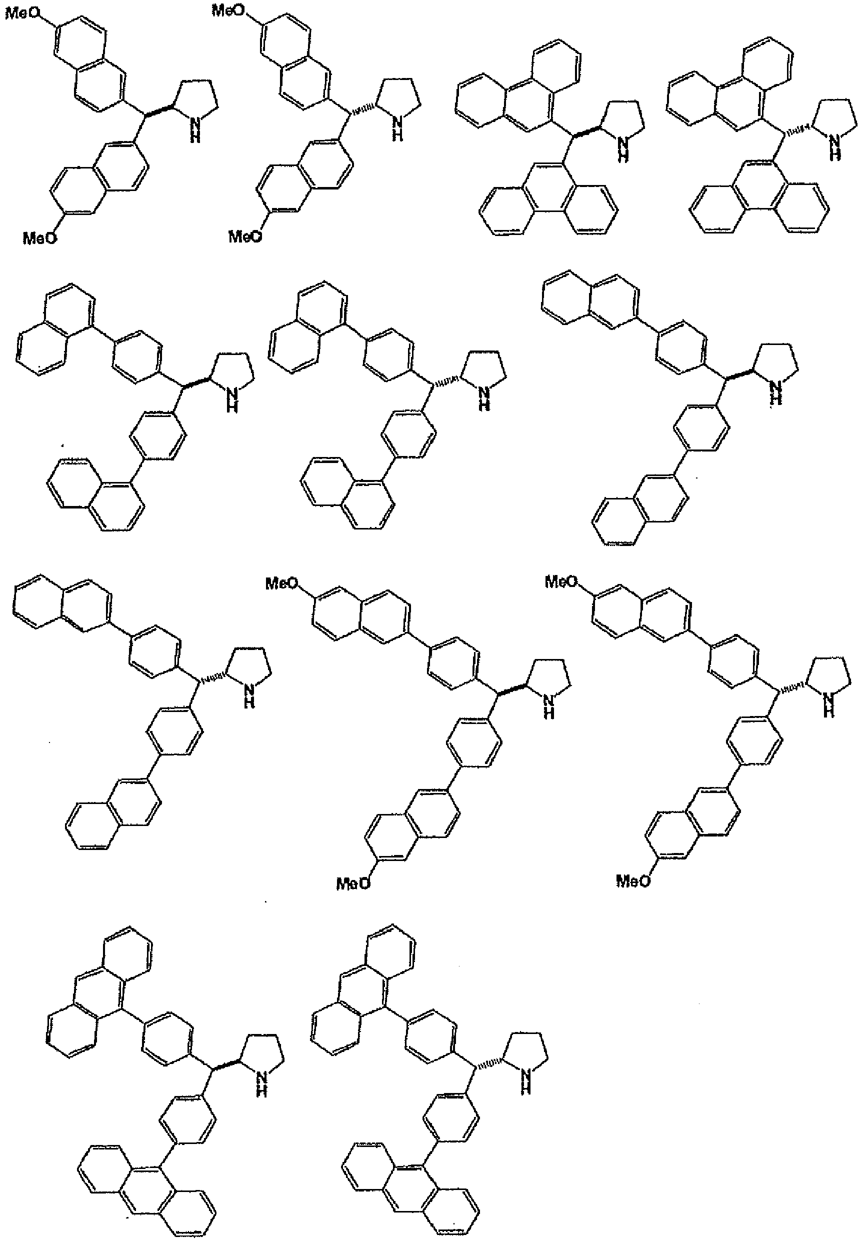

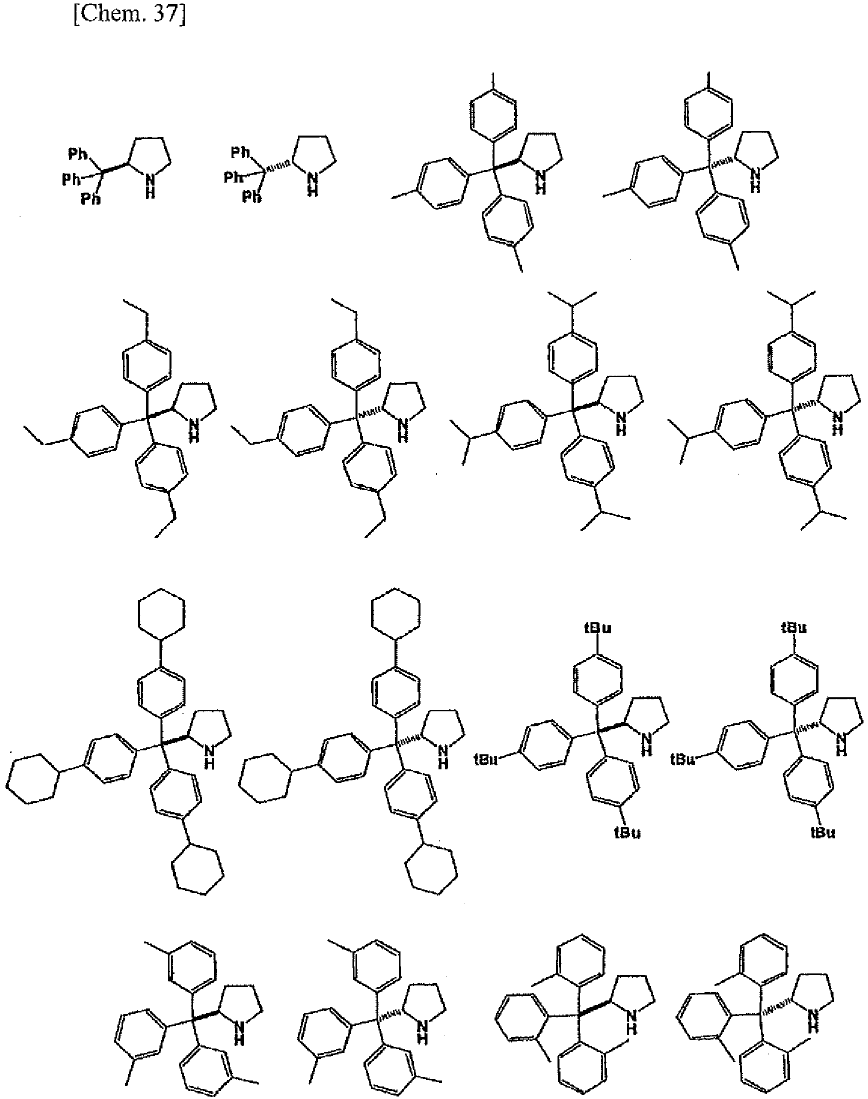

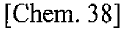

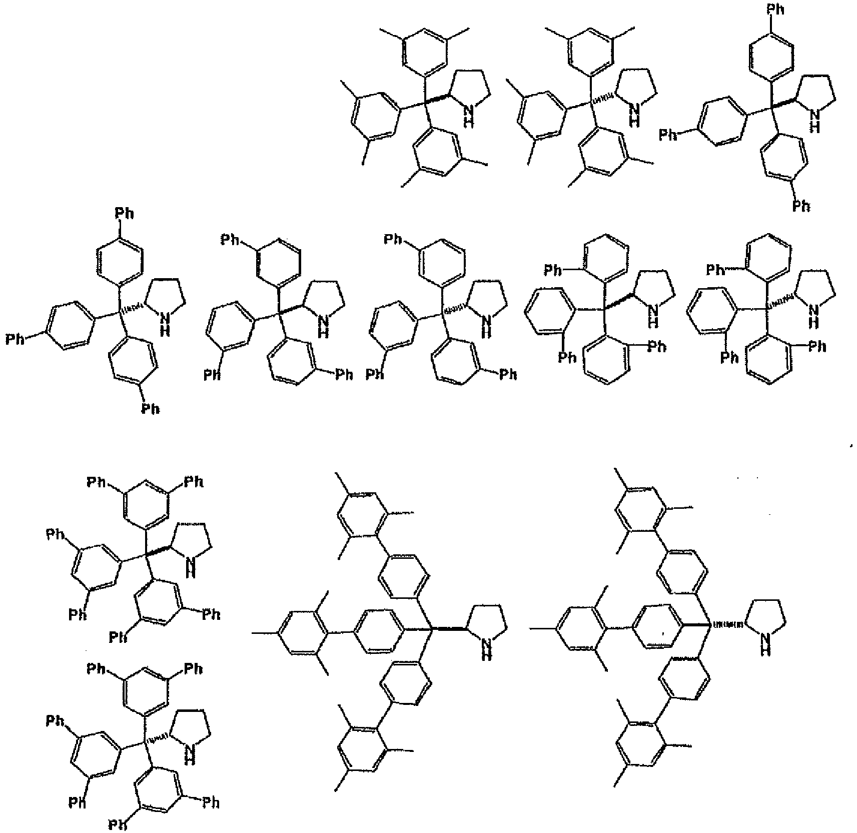

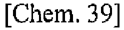

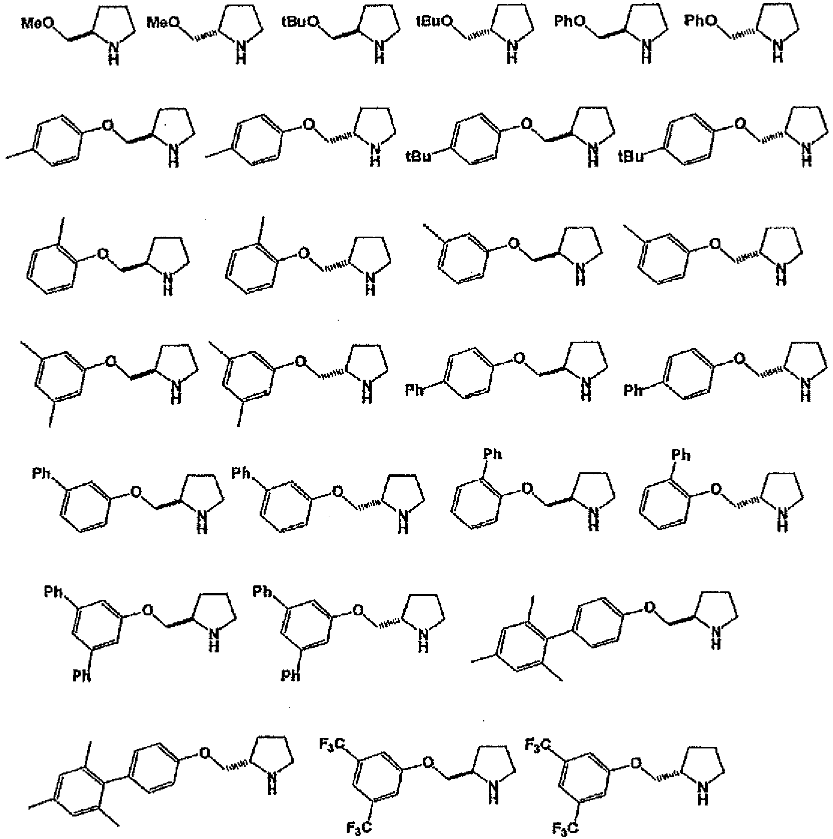

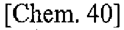

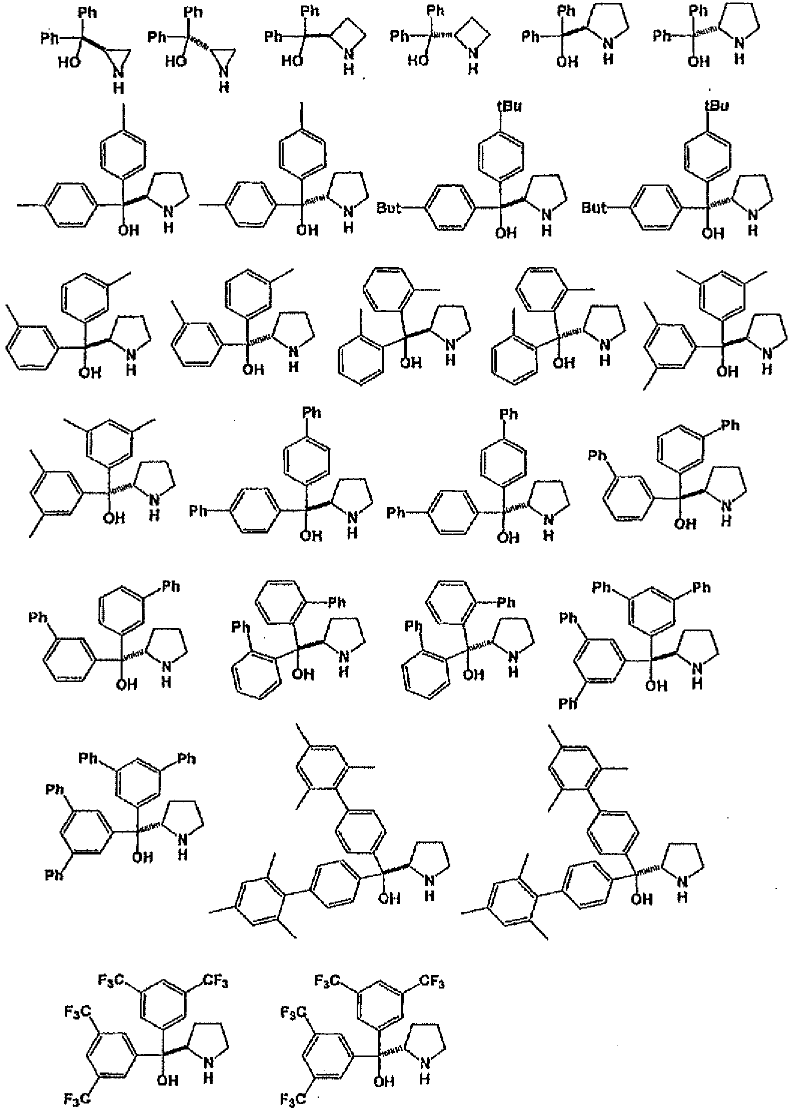

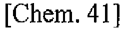

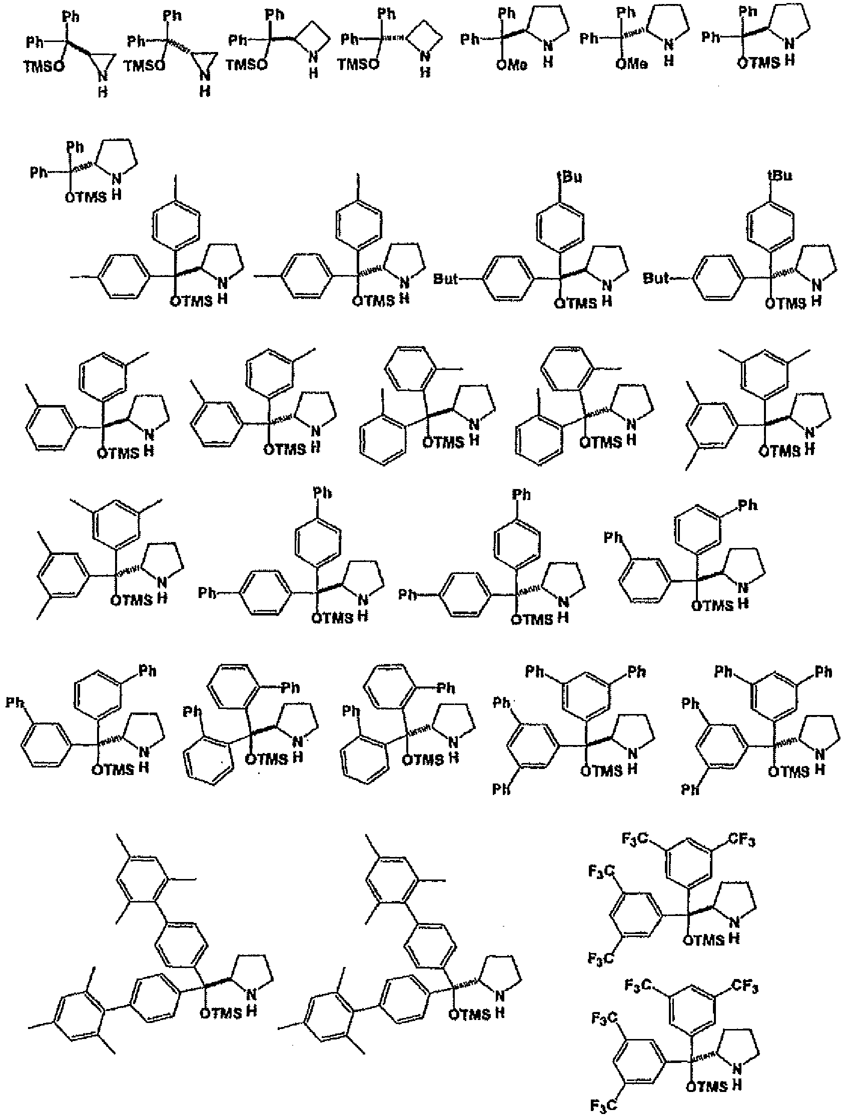

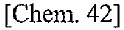

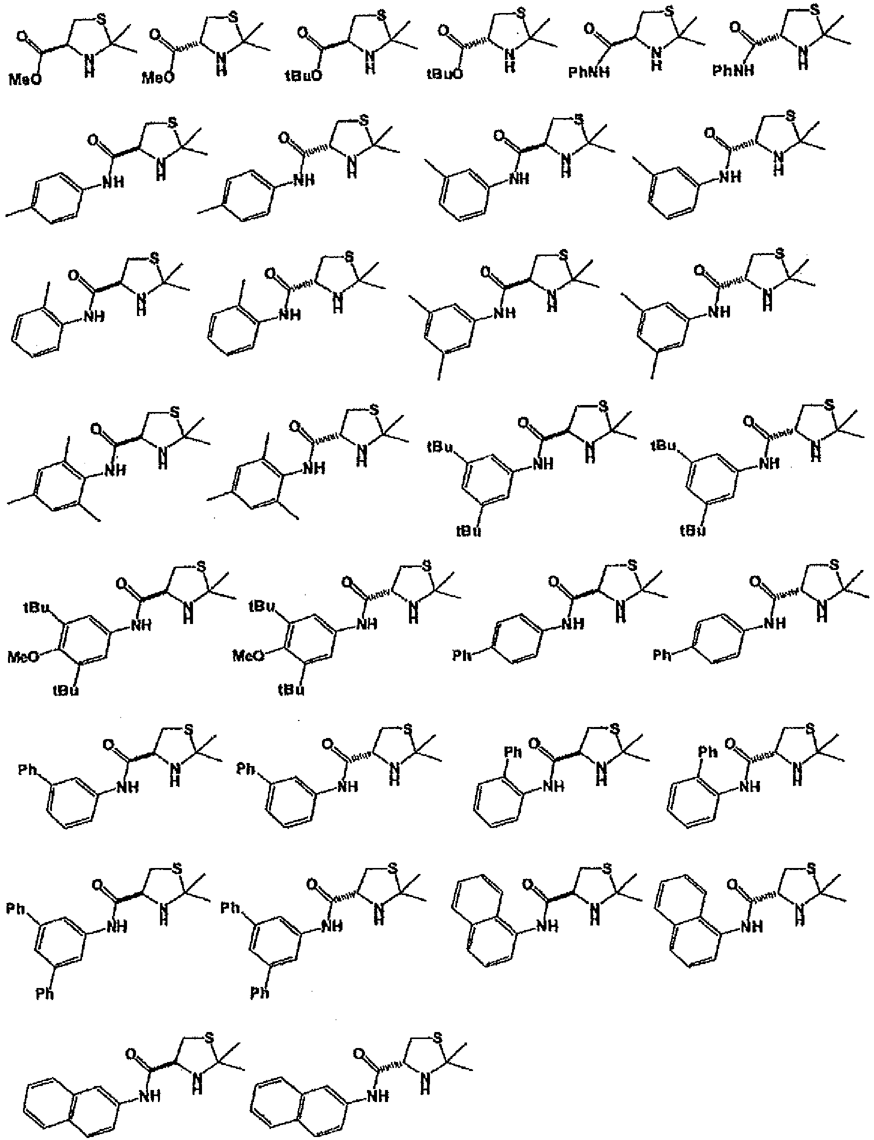

In addition, in the present invention, it is preferable that an acid is used as another catalyst component. By using an acid, the optically active cyclic nitrogen-containing compound can present as a salt tereof in a system and catalyst reaction can be thus facilicated. In addition, the acid is present as a counter of the complex catalyst, and stabilization of the complex catalyst can be thus realized.
The acid may be an organic acid or an inorganic acid and is particularly preferable an organic acid.

As illustrative examples of the organic acid, there will be mentioned acetic acid, chloroacetic acid, difluoroacetic acid, trifluoroacetic acid, trichloroacetic acid, tribromoacetic acid, benzoic acid, 2,4-dinitrobenzoic acid, p-toluenesulfonic acid, methanesulfonic acid, trifluoromethanesulfonic acid and the like.
As illustrative examples of the inorganic acid, there will be mentioned hydrofluoric acid, hydrochloric acid, hydrobromic acid, hydroiodic acid, sulfuric acid, perchloric acid, phosphoric acid, nitric acid and the like.

In the case where the transition metal complex reacts with the optically active cyclic nitrogen-containing compound, and reacts with an acid or auxiliary ligand, if necessary, to previously prepare a catalyst, the transition metal complex, the nitrogen compound, and the acid or auxiliary ligand, if necessary, may be added to a solvent thereby preparing the catalyst in situ, or the solvent is distilled away from the solution in which the catalyst has been previously prepared in the solvent and the solution is then added to the target hydrogenation substrate. Through this process, the catalyst for homogeneous asymmetric hydrogenation of the presnt invention can be obtained.

### <Preparation method>

In the present invention, an optically active carbonyl compound such as optically active aldehyde or optically active ketone can be obtained by subjecting α, β-unsaturated carbonyl compounds to asymmetric hydrogenation reaction in the presence of the catalyst, or in the presence of the transition metal complex and the optically active cyclic nitrogen-containing compound. Furthermore, respective compounds including the transition metal complex and optically active cyclic nitrogen-containing compound can be commercially available.

The amount of transition metal complex used for the catalyst of the present invention depends on various reaction conditions, but is for example 0.0001 to 1 time by mol and is preferably 0.001 to 0.05 times by mol based on the α, β-unsaturated carbonyl compound, as the substrate..

The amount of optically active cyclic nitrogen-containing compound used for the catalyst component of the present invention depends on various reaction conditions, but is for example 0.0001 to 20 times by mol and is preferably 0.001 to 0.05 times by mol based on the α, β-unsaturated carbonyl compound, as the substrate.

Using amount of the acid to be used as catalyst components of the present invention vary depending on various reaction conditions, but is, for example, 0.01 to 10 times by mol, preferably 0.2 to 4 times by mol based on the optically active cyclic nitrogen-containing compound.

When an optically active carbonyl compound is produced by carrying out asymmetric hydrogenation of the α,β-unsaturated carbonyl compound using the catalyst of the present invention, it can be carried out in the presence or absence of a solvent, but it is desirable to carry it out in the presence of a solvent.

As the illustrative solvent to be used, aliphatic hydrocarbon-based organic solvents such as hexane, heptane and octane; alicyclic hydrocarbon-based organic solvents such as cyclohexane and methylcyclohexane; aromatic hydrocarbon-based organic solvents such as benzene, toluene and xylene; ether-based organic solvents such as diethyl ether, diisopropyl ether, dimethoxyethane, tetrahydrofuran, dioxane and dioxolan; water; alcohol-based organic solvents such as methanol, ethanol, propanol, isopropanol and tertiary butanol; halogenated hydrocarbon-based organic solvents such as dichloromethane, dichloroethane, chlorobenzene and bromotoluene; dimethylformamide, acetonitrile and the like are desirable, and a mixed solvent of these solvents can also be used in response to the necessity. Among these solvents, heptane, toluene and tetrahydrofuran are particularly desirable.
Using amount of the solvent can be optionally selected based on the reaction conditions and the like, but is, for example, from 0 to 20 times volume, preferably from 0 to 5 times volume, based on the α,β-unsaturated carbonyl compound as the substrate.

The method of the present invention is carried out using hydrogen gas as the hydrogen source, and its hydrogen pressure is from 0.01 MPa to 10 MPa, preferably from 0.1 MPa to 1 MPa. The reaction temperature is from -78 to 100°C, preferably from 10 to 40°C. The reaction time varies depending on the reaction conditions, but is generally from 1 to 30 hours.

The optically active carbonyl compound obtained as described in the above can be isolated and purified by generally used operations such as extraction, recrystallization, various types of chromatography and the like. In addition, regarding configuration of the thus obtained optically active carbonyl compound, its d-form or 1-form (R-form or S-form) can be produced by optionally selecting configuration of the optically active cyclic nitrogen-containing compound.

### Examples

Hereinafter, the present invention will be described in detail with reference to examples and comparative examples. The present invention is not limited to these examples.

Furthremore, the measurmerent of products in Examples was carried out using the following apparatuses.
Neuclear magnetic resonance spectrum :
¹H-NMR : Oxford 300 MHz FT-NMR (300 MHz)( manufactured by Varian Medical Systems, Inc.)
Gas chromatography :
GC-2010 gas chromatography (manufactured by Shimadzu Corp.)
Column :
Measurement of conversion ratio: DB-WAX (0.25 mm × 30 m) (manufactured by Agilent Corp.)
Measurement of optical purity: β-DEX-225 (0.25 mm × 30 m), β-DEX-325 (0.25 mm × 30 m) (manufactured by SUPELCO Corp.)
Detector : FID
In addition, the metal complex was commercially available from Aldrich Corp. or Strem Corp. and the optically active cyclic nitrogen-containing compound was commercially available from Aldrich Corp.

### (Examples 1-1 to 1-15)

An example in which a palladium complex is used as a catalyst metal and (R)-(+)-2-(diphenylmethyl) pyrrolidine is used as a cocatalyst (optically active cyclic nitrogen-containing compound) will be described below.

(R)-(+)-2-(diphenylmethyl) pyrrolidine

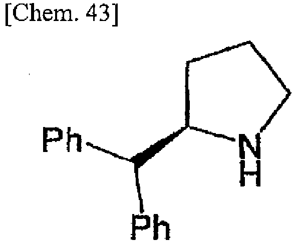

### (Example 1-1)

1g of citral (6.57 mmol, a ratio of neral to geranial in a reagent = 1 : 1), 2.9 mg of palladium acetate (0.2 mol%), 31 mg of (R)-(+)-2-(diphenylmethyl) pyrrolidine (2 mol%), 15 mg of trifluoroacetic acid (2 mol%), and 2 ml of toluene were added to a 10 ml reaction flask, followed by stirring under a hydrogen atmosphere. The reaction mixture was stirred at room temperature for 21 hours and the analysis thereof was performed by gas chromatography. As a result, a conversion ratio from citral to citronellal was 50.6%, the obtained citronellal was a d-form and an optical purity thereof was 40.3% e.e.

### (Example 1-2)

1g of citral (6.57 mmol, a ratio of neral to geranial in a reagent = 1 : 1), 5.8 mg of palladium acetate (0.4 mol%), 31 mg of (R)-(+)-2-(diphenylmethyl) pyrrolidine (2 mol%), and 2 ml of toluene were added to a 10 ml reaction flask, followed by stirring under a hydrogen atmosphere. The reaction mixture was stirred at room temperature for 21 hours and the analysis thereof was performed by gas chromatography. As a result, a conversion ratio from citral to citronellal was 99.8%, the obtained citronellal was a d-form and an optical purity thereof was 18.2% e.e.

### (Example 1-3)

1g of neral (6.57 mmol), 5.8 mg of palladium acetate (0.4 mol%), 31 mg of (R)-(+)-2-(diphenylmethyl) pyrrolidine (2 mol%), 15 mg of trifluoroacetic acid (2 mol%), and 2 ml of toluene were added to a 10 ml reaction flask, followed by stirring under a hydrogen atmosphere. The reaction mixture was stirred at room temperature for 21 hours and the analysis thereof was performed by gas chromatography. As a result, a conversion ratio from neral to citronellal was 94.5%, the obtained citronellal was a d-form and an optical purity thereof was 25.6% e.e.

### (Example 1-4)

1g of geranial (6.57 mmol), 5.8 mg of palladium acetate (0.4 mol%), 31 mg of (R)-(+)-2-(diphenylmethyl) pyrrolidine (2 mol%), 15 mg oftrifluoroacetic acid (2 mol%), and 2 ml of toluene were added to a 10 ml reaction flask, followed by stirring under a hydrogen atmosphere. The reaction mixture was stirred at room temperature for 21 hours and the analysis thereof was performed by gas chromatography. As a result, a conversion ratio from geranial to citronellal was 93.2%, the obtained citronellal was a d-form and an optical purity thereof was 40.5% e.e.

### (Examples 1-5 to 1-15)

A reaction was performed in the same manner as in Example 1 except that the palladium complex, the solvent and the auxiliary ligand were changed. The results thus obtained are shown in Table 1 below. All of the obtained citronellal were a d-form.

**[Table 1]**

| Example | Pd complex | Auxiliary ligand | | Solvent | Conversion ratio (%) | Optical purity (%ee) |
|---|---|---|---|---|---|---|
| | | Type | Added amount | | | |
| 1-5 | Pd(OAc)₂ | - | - | THF | 67.6 | 49.8 |
| 1-6 | Pd(OAc)₂ | - | - | MeCN | 15.0 | 64.3 |
| 1-7 | Pd(OAc)₂ | 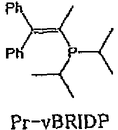 | 8 mol% | Tol | 50.3 | 25.9 |
| 1-8 | Pd(OAc)₂ | Tri-n-octyl phosphine | 8 mol% | Tol | 2.50 | 17.1 |
| 1-9 | Pd(OAc)₂ | 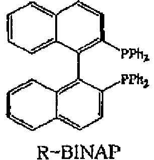 | 4 mol% | Tol | 28.1 | 60.0 |
| 1-10 | Pd(OAc)₂ | 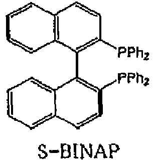 | 4 mol% | Tol | 38.3 | 60.7 |
| 1-11 | Pd(OAc)₂ | 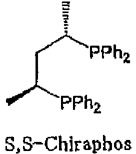 | 4 mol% | Tol | 72.1 | 47.5 |
| 1-12 | Pd(dba)₂ | - | - | Tol | 30.5 | 46.8 |
| 1-13 | Pd(dba)₂ | 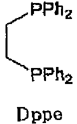 | 8 mol% | Tol | 30.0 | 51.5 |
| 1-14 | [π-allylPdCl]₂ | - | - | Tol | 98.3 | 70.0 |
| 1-15 | [π-allylPdCl]₂ | - | - | MeCN | 12.3 | 48.8 |

### (Examples 2-1 to 2-3)

Preparation Example of the catalyst and Examples of asymmetric hydrogenation reaction using the prepared catalyst will be described below.

### (Example 2-1)

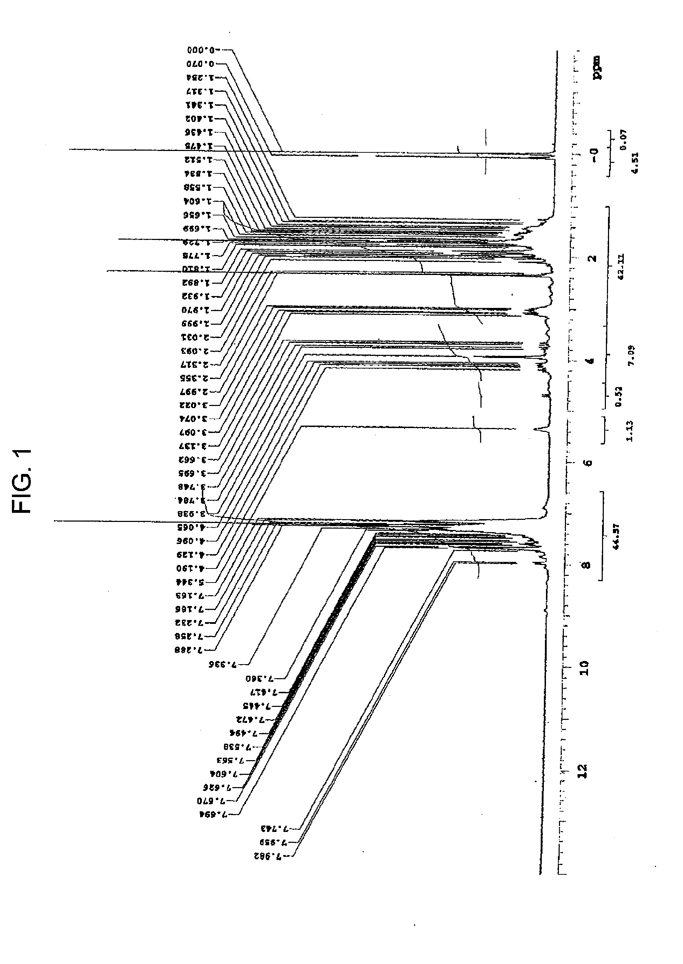

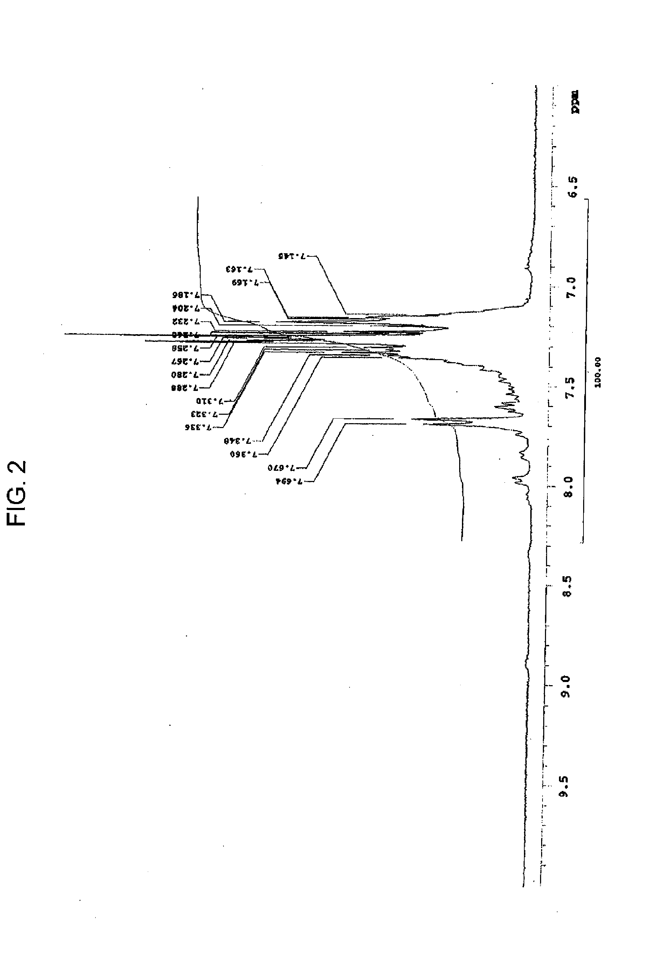

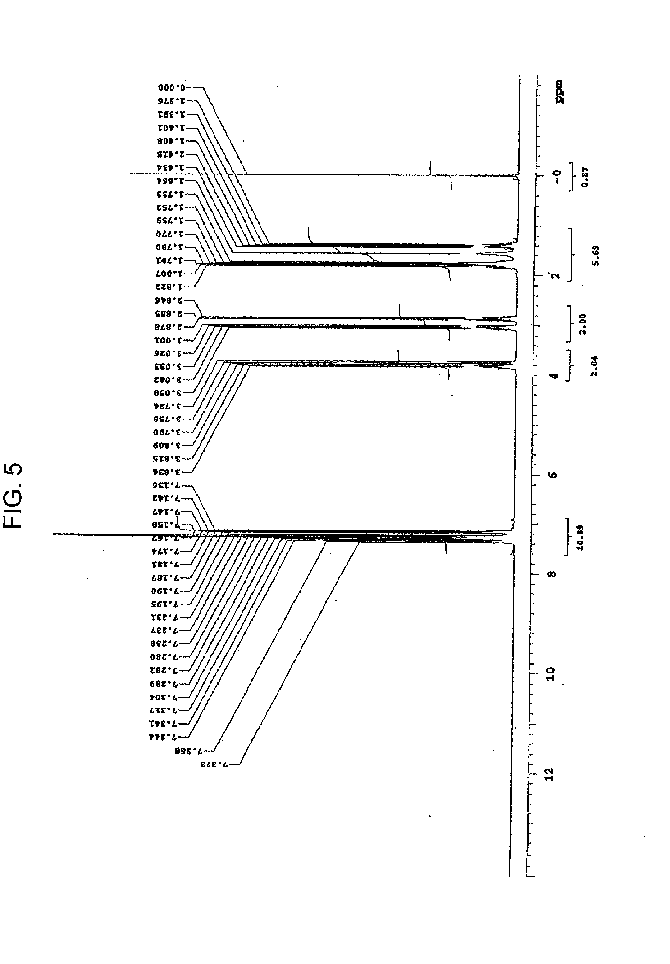

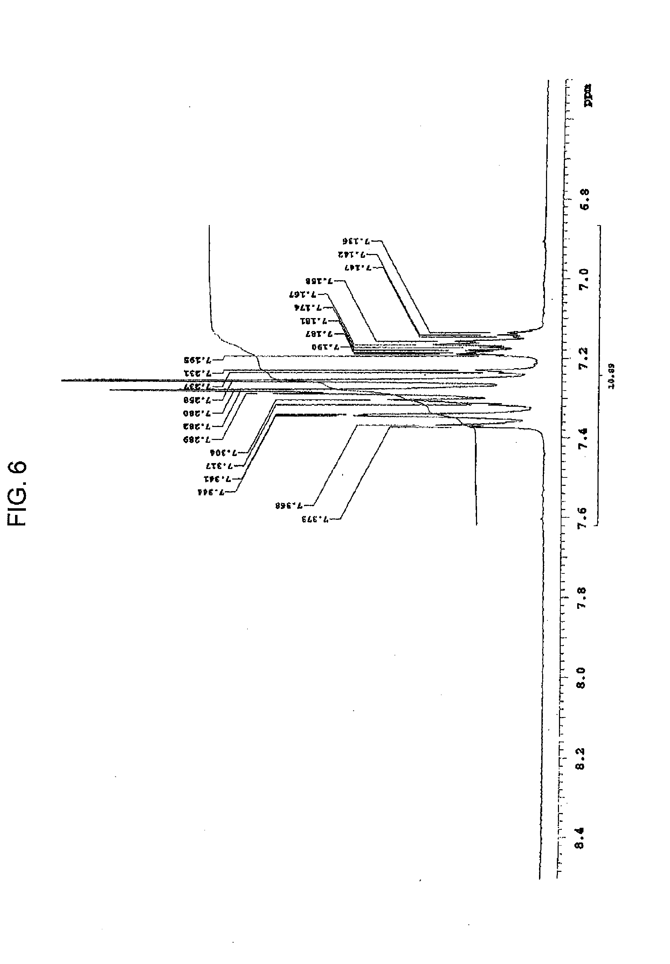

2.9 mg of palladium acetate (0.2 mol%), 31 mg of (R)-(+)2-(diphenylmethyl) pyrrolidine (2 mol%), and 2 ml of toluene were added to a 10 ml reaction flask. After degassing, the reaction mixture was stirred at room temperature one night and then concentrated to obtain a complex.
The fact that this complex had a structure in which (R)-(+)-2-(diphenylmethyl) pyrrolidine was coordinated to palladium was confirmed from the viewpoint that (R)-(+)-2-(diphenylmethyl) pyrrolidine and other signals were observed on H¹-NMR.
The H¹-NMR chart of the complex is shown in FIGs. 1 and 2. In addition, as a control, the H¹-NMR chart of (R)-(+)-2-(diphenylmethyl) pyrrolidine is shown in FIGs. 5 and 6.

### (Example 2-2)

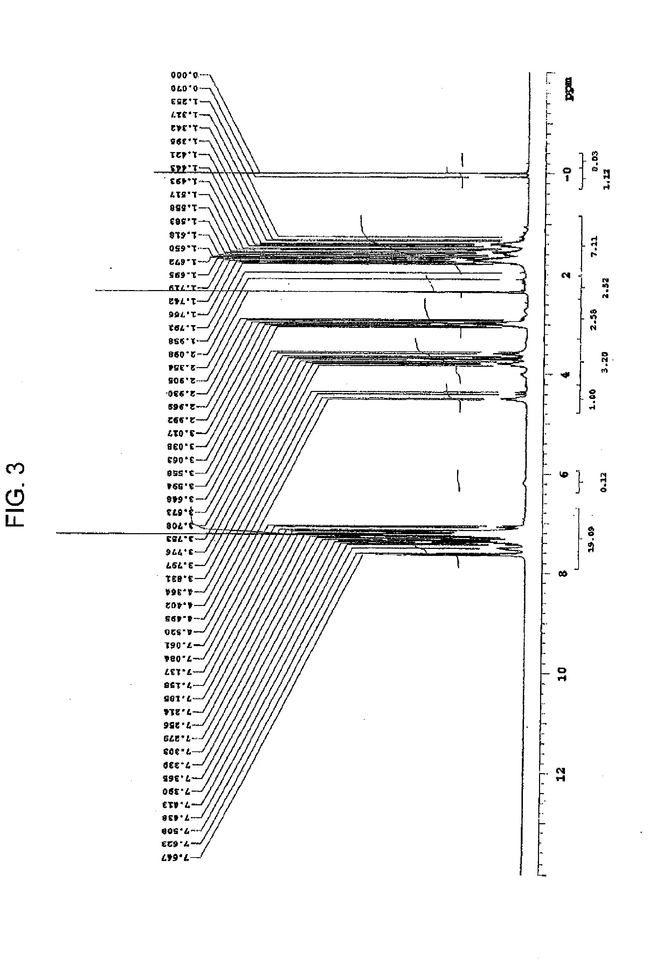

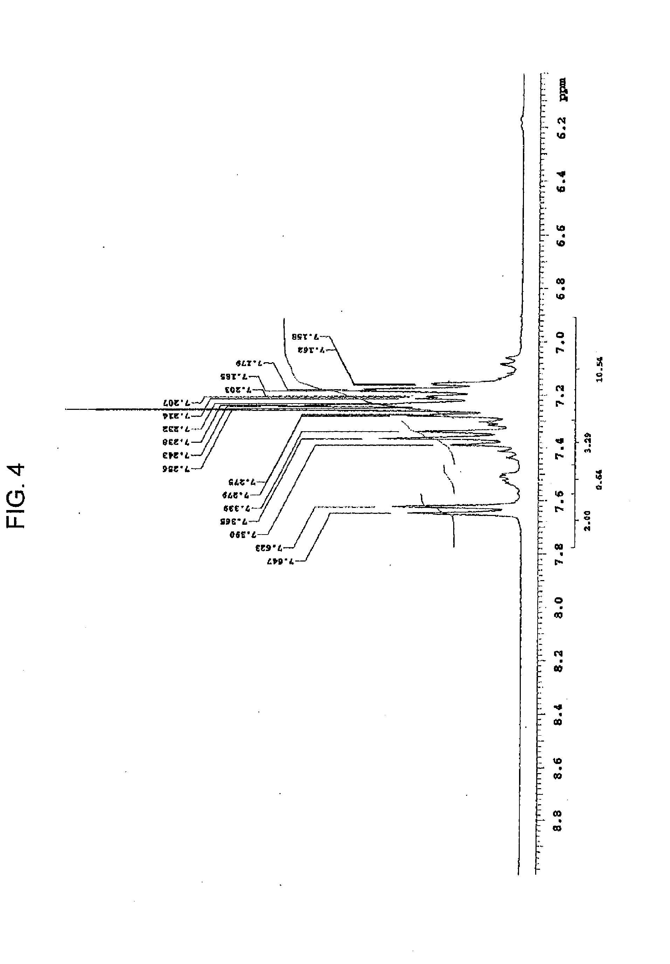

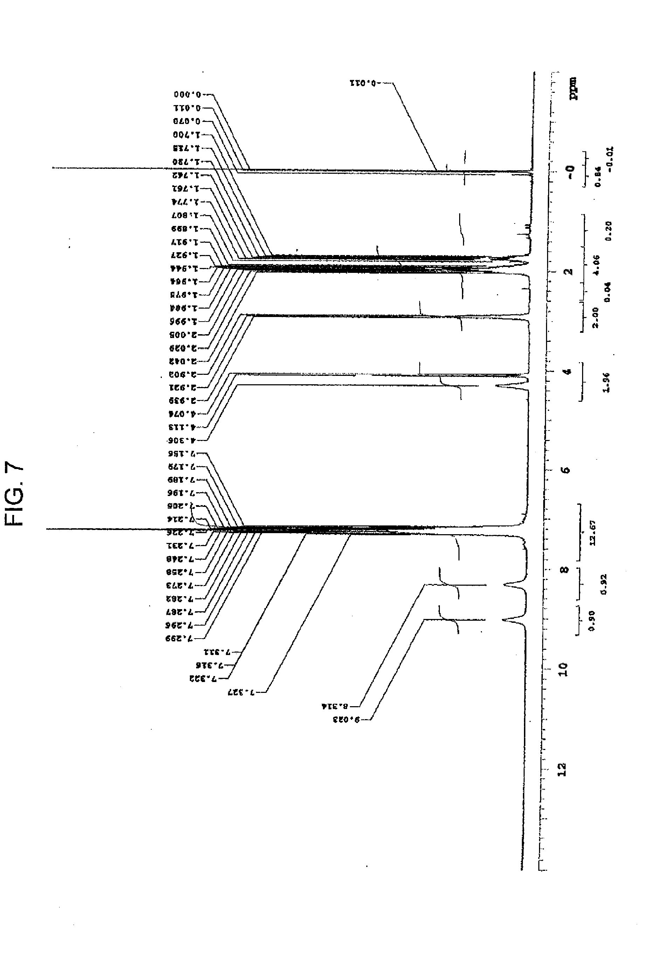

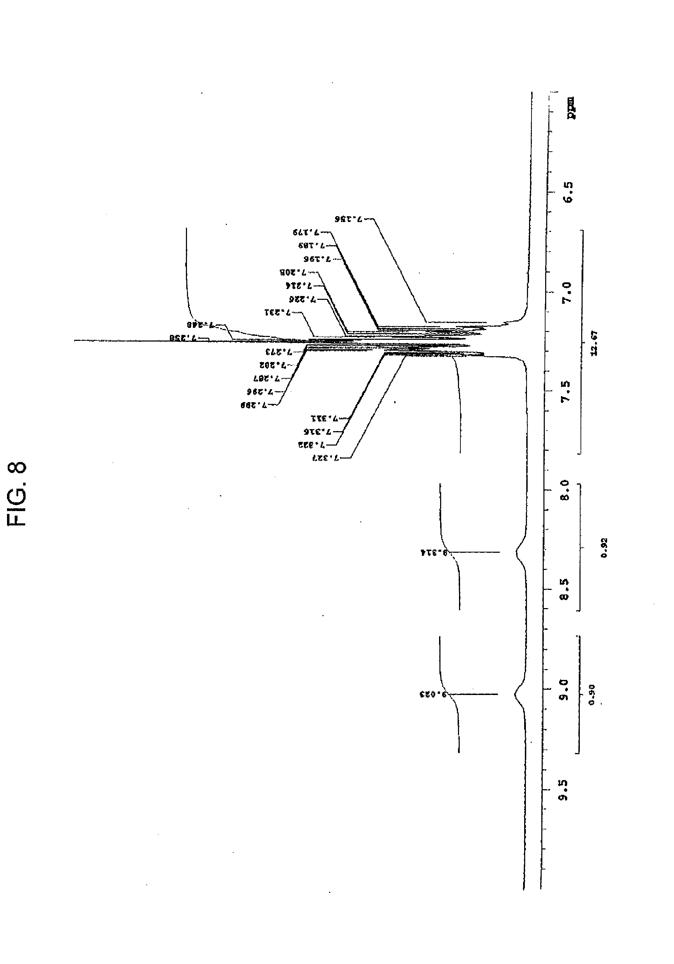

2.9 mg of palladium acetate (0.2 mol%), 31 mg of (S)-(+)-2-(diphenylmethyl) pyrrolidine (2 mol%), 15 mg of trifluoroacetic acid (2 mol%), and 2 ml of toluene were added to a 10 ml reaction flask. After degassing, the reaction mixture was stirred at room temperature one night and then concentrated to obtain a complex.
The fact that this complex had a structure in which (R)-(+)-2-(diphenylmethyl) pyrrolidine was coordinated to palladium was confirmed from the viewpoint that (R)-(+)-2-(diphenylmethyl) pyrrolidine and other signals were observed on H¹-NMR.
The H¹-NMR chart of the complex is shown in FIGs. 3 and 4. In addition, as a control, the H¹-NMR chart of (R)-(+)-2-(diphenylmethyl) pyrrolidine is shown in FIGs. 5 and 6, and the H¹-NMR chart of trifluoroacetic acid salt of (R)-(+)-2-(diphenylmethyl) pyrrolidine is shown in Figs. 7 and 8.

### (Example 2-3)

1g of citral (6.57 mmol, a ratio of neral to geranial in a reagent = 1 : 1), the catalyst obtained in Example 2-1, 15 mg of trifluoroacetic acid (2 mol%), and 2 ml of toluene were added to a 10 ml reaction flask, followed by stirring under a hydrogen atmosphere. The reaction mixture was stirred at room temperature for 21 hours and the analysis thereof was performed by gas chromatography. As a result, a conversion ratio from citral to citronellal was 50.6%, the obtained citronellal was a d-form and an optical purity thereof was 40.3% e.e.

### (Examples 3-1 to 3-4)

An example in which (S)-(+)-2-(tert-butyl)-3-methyl-4-imidazolidinone trifluoroacetic acid salt is used as a cocatalyst (optically active cyclic nitrogen-containing compound) will be described below.

(S)-(+)- 2-(tert-butyl)-3-methyl-4-imidazolidinone trifluoroacetic acid salt

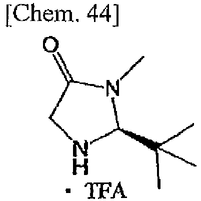

### (Example 3-1)

1g of citral (6.57 mmol, a ratio of neral to geranial in a reagent = 1 : 1), 5.8 mg of palladium acetate (0.4 mol%), 36 mg of (S)-(+)-2-(tert-butyl)-3-methyl-4-imidazolidinone trifluoroacetic acid salt (2 mol%) and 2 ml of toluene were added to a 10 ml reaction flask, followed by stirring under a hydrogen atmosphere. The reaction mixture was stirred at room temperature for 21 hours and the analysis thereof was performed by gas chromatography. As a result, a conversion ratio from citral to citronellal was 51.0%, the obtained citronellal was an 1-form and an optical purity thereof was 27.1% e.e.

### (Example 3-2)

1g of citral (6.57 mmol, a ratio of neral to geranial in a reagent = 1 : 1), 5.8 mg of palladium acetate (0.4 mol%), 36 mg of (S)-(+)-2-(tert-butyl)-3-methyl-4-imidazolidinone trifluoroacetic acid salt (2 mol%) and 2 ml of THF were added to a 10 ml reaction flask, followed by stirring under a hydrogen atmosphere. The reaction mixture was stirred at room temperature for 21 hours and the analysis thereof was performed by gas chromatography. As a result, a conversion ratio from citral to citronellal was 58.5%, the obtained citronellal was an 1-form and an optical purity thereof was 34.8% e.e.

### (Example 3-3)

1 g of citral (6.57 mmol, a ratio of neral to geranial in a reagent = 1 : 1), 16.4 mg of R-BINAP (0.4 mol%), 5.8 mg of palladium acetate (0.4 mol%), 36 mg of (S)-(+)-2-(tert-butyl)-3-methyl-4-imidazolidinone trifluoroacetic acid salt (2 mol%), and 2 ml of THF were added to a 10 ml reaction flask, followed by stirring under a hydrogen atmosphere. The reaction mixture was stirred at room temperature for 21 hours and the analysis thereof was performed by gas chromatography. As a result, a conversion ratio from citral to citronellal was 10.0%, the obtained citronellal was an 1-form and an optical purity thereof was 29.5% e.e.

### (Example 3-4)

1g of citral (6.57 mmol, a ratio of neral to geranial in a reagent = 1 : 1), 5.8 mg of palladium acetate (0.4 mol%), 25 mg of (L)-2-(diphenylamido) pyrrolidine (2 mol%), 15 mg of trifluoroacetic acid (2 mol%), and 2 ml of toluene were added to a 10 ml reaction flask, followed by stirring under a hydrogen atmosphere. The reaction mixture was stirred at room temperature for 21 hours and the analysis thereof was performed by gas chromatography. As a result, a conversion ratio from citral to citronellal was 41.9%, the obtained citronellal was an 1-form and an optical purity thereof was 3.0% e.e.

### (Examples 4-1 to 4-4)

Examples in which the following Willkinson complex, the following pentamethylcyclopentadienyl iridium dichloride dimer or trichloroiridium n-hydrate is used as a transition metal complex, and (R)-(+)-2-(diphenylmethyl) pyrrolidine is used as a cocatalyst (optically active cyclic nitrogen-containing compound) will be described below.

Willkinson complex

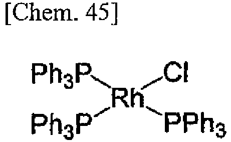

Pentamethylcyclopentadienyl iridium dichloride dimer

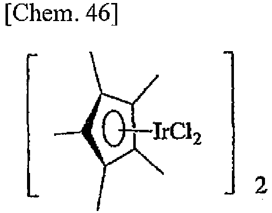

### (Example 4-1)

1g of citral (6.57 mmol, a ratio of neral to geranial in a reagent = 1 : 1), 62.8 mg of a Willkinson complex (1 mol%), 31 mg of (R)-(+)-2-(diphenylmethyl) pyrrolidine (2 mol%), 15 mg of trifluoroacetic acid (2 mol%), and 2 ml of ethanol were added to a 10 ml reaction flask, followed by stirring under a hydrogen atmosphere. The reaction mixture was stirred at room temperature for 21 hours and the analysis thereof was performed by gas chromatography. As a result, a conversion ratio from citral to citronellal was 28.7%, the obtained citronellal was an d-form and an optical purity thereof was 5.0% e.e.

### (Example 4-2)

1g of citral (6.57 mmol, a ratio of neral to geranial in a reagent = 1 : 1), 62.8 mg of Willkinson complex (1 mol%), 31 mg of (R)-(+)-2-(diphenylmethyl) pyrrolidine (2 mol%), and 2 ml of ethanol were added to a 10 ml reaction flask, followed by stirring under a hydrogen atmosphere. The reaction mixture was stirred at room temperature for 21 hours and the analysis thereof was performed by gas chromatography. As a result, a conversion ratio from citral to citronellal was 65.7%, the obtained citronellal was a d-form and an optical purity thereof was 1.5% e.e.

### (Example 4-3)

1g of citral (6.57 mmol, a ratio of neral to geranial in a reagent = 1 : 1), 19.5 mg of trichlorideiridium n-hydrate (2 mol%), 31 mg of (R)-(+)-2-(diphenylmethyl) pyrrolidine (2 mol%), 15 mg of trifluoroacetic acid (2 mol%) and 2 ml of THF were added to a 10 ml reaction flask, followed by stirring under a hydrogen atmosphere. The reaction mixture was stirred at room temperature for 21 hours and the analysis thereof was performed by gas chromatography. As a result, a conversion ratio from citral to citronellal was 9.02%, the obtained citronellal was a d-form and an optical purity thereof was 20.8% e.e.

### (Example 4-4)

1g of citral (6.57 mmol, a ratio of neral to geranial in a reagent = 1 : 1), 19.6 mg of pentamethylcyclopentadienyl iridium dichloride dimer (1 mol%), 31 mg of (R)-(+)-2-(diphenylmethyl) pyrrolidine (2 mol%), 15 mg of trifluoroacetic acid (2 mol%) and 2 ml of THF were added to a 10 ml reaction flask, followed by stirring under a hydrogen atmosphere. The reaction mixture was stirred at room temperature for 21 hours and the analysis thereof was performed by gas chromatography. As a result, a conversion ratio from citral to citronellal was 11.2%, the obtained citronellal was a d-form and an optical purity thereof was 3.9% e.e.

### (Examples 5-1 to 5-3)

An example in which a hexarhodium hexadecacarbonyl complex is used as a transition metal complex, (+)-2,3-O-isopropylidene-2,3-dihydroxy-1,4-bis(diphenylphosphino) butane (hereinafter, referred to as (+)-DIOP) is used as a ligand, and a (S)-(+)-2-(tert-butyl)-3-methyl-4-imidazolidinone trifluoroacetic acid salt is used as a cocatalyst (optically active cyclic nitrogen-containing compound) will be described below.

(+)-DIOP

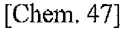

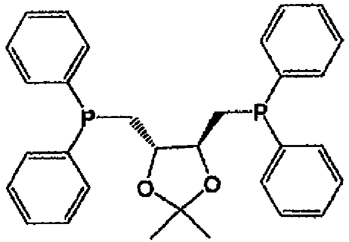

### (Example 5-1)

550 mg of geranial (3.6 mmol), 5.4 mg of a hexarhodium hexadecacarbonyl complex (0.83 mol%), 19.5 mg of a (S)-(+)-2-(tert-butyl)-3-methyl-4-imidazolidinone trifluoroacetic acid salt (2 mol%), 37.6 mg of (+)-DIOP (2.1 mol%) and 2 ml of toluene were added to a 10 ml reaction flask, followed by stirring under a hydrogen atmosphere. The reaction mixture was stirred at room temperature for 21 hours and the analysis thereof was perfomed by gas chromatography. As a result, a conversion ratio from geranial to citronellal was 29.8%, the obtained citronellal was a d-form and an optical purity thereof was 4.5% e.e.

### (Example 5-2)

550 mg of neral (3.6 mmol), 5.4 mg of a hexarhodium hexadecacarbonyl complex (0.83 mol%), 19.5 mg of a (S)-(+)-2-(tert-butyl)-3-methyl-4-imidazolidinone trifluoroacetic acid salt (2 mol%), 37.6 mg of (+)-DIOP (2.1 mol%) and 2 ml of toluene were added to a 10 ml reaction flask, followed by stirring under a hydrogen atmosphere. The reaction mixture was stirred at room temperature for 21 hours and the analysis thereof was perfomed by gas chromatography. As a result, a conversion ratio from neral to citronellal was 32.6%, the obtained citronellal was a d-form and an optical purity thereof was 4.3% e.e.

### (Example 5-3)

550 mg of citral (3.6 mmol, a ratio of neral to geranial in a reagent = 1 : 1), 5.4 mg of a hexarhodium hexadecacarbonyl complex (0.83 mol%), 19.5 mg of a (S)-(+)-2-(tert-butyl)-3-methyl-4-imidazolidinone trifluoroacetic acid salt (2 mol%), 37.6 mg of (+)-DIOP (2.1 mol%) and 2 ml of toluene were added to a 10 ml reaction flask, followed by stirring under a hydrogen atmosphere. The reaction mixture was stirred at room temperature for 21 hours and the analysis thereof was perfomed by gas chromatography. As a result, a conversion ratio from citral to citronellal was 34.9%, the obtained citronellal was a d-form and an optical purity thereof was 4.8% e.e.

### (Comparative Examples 1-1 to 1-2)

Examples in which a reaction is performed using a hexarhodium hexadecacarbonyl complex as a transition metal complex, using (+)-2,3-O-isopropylidene-2,3-dihydroxy-1,4-bis(diphenylphosphino) butane (hereinafter, referred to (+)-DIOP) as a ligand, and using no cocatalyst (optically active cyclic nitrogen-containing compound) will be described below.

### (Comparative Example 1-1)

550 mg of geranial (3.6 mmol), 5.4 mg of a hexarhodium hexadecacarbonyl complex (0.83 mol%), 37.6 mg of (+)-DIOP (2.1 mol%) and 2 ml of toluene were added to a 10 ml reaction flask, followed by stirring under a hydrogen atmosphere. The reaction mixture was stirred at room temperature for 21 hours and the analysis thereof was performed by gas chromatography. As a result, a conversion ratio from geranial to citronellal was 95.5%, the obtained citronellal was an 1-form and an optical purity thereof was 48.6% e.e.

### (Comparative Example 1-2)

550 mg of neral (3.6 mmol), 5.4 mg of a hexarhodium hexadecacarbonyl complex (0.83 mol%), 37.6 mg of (+)-DIOP (2.1 mol%) and 2 ml of toluene were added to a 10 ml reaction flask, followed by stirring under a hydrogen atmosphere. The reaction mixture was stirred at room temperature for 21 hours and the analysys thereof was performed by gas chromatography. As a result, a conversion ratio from neral to citronellal was 97.1%, the obtained citronellal was a d-form and an optical purity thereof was 47.1% e.e.

As apparent from Comparative Examples 1-1 to 1-2, when the reaction is performed without using any cocatalyst (optically active cyclic nitrogen-containing compound), steric configuration of the obtained product depends on Z-configuration or E-configuration of the starting substrate.

While the present invention has been described in detail and with reference to specific embodiments thereof, it will be apparent to one skilled in the art that various changes and modifications can be made therein without departing from the spirit and scope thereof. This application is based on Japanese Patent Application No. 2010-047741 filed on March 4, 2010, and their contents are incorporated herein by reference.

### Industrial Applicability

The optically active α, β-carbonyl compound can be manufactured by selectively and easily asymmetrically hydrogenating the carbon-carbon double bond of the α, β-unsaturated carbonyl compound, as a substrate, with the use of the catalyst for asymmetric hydrogenation of the present invention which is prepared by simply mixing: a specific metal complex and an optically active cyclic nitrogen-containing compound; or a specific metal complex, an optically active cyclic nitrogen-containing compound and an acid. In particular, optically active citronellal can be obtained by selectively asymmetrically hydrogenating α, β-carbon-carbon double bonds of citral (mixture of geranial and neral), geranial, or neral. The optically active citronellal itself is useful as a flavoring and is an important raw material of optically active citronellol, optically active isopulegol and optically active menthol.

## Claims

1. A catalyst for homogenous asymmetric hydrogenation, comprising:
a complex of at least one transition metal selected from transition metals belonging to Group 8 to Group 10 of the Periodic Table; and
an optically active cyclic nitrogen-containing compound.

2. The catalyst for homogenous asymmetric hydrogenation according to claim 1, which is formed by coordinating the the optically active cyclic nitrogen-containing compound to the at least one transition metal selected from transition metals belonging to Group 8 to Group 10 of the Periodic Table.

3. A catalyst for homogenous asymmetric hydrogenation, comprising:
a complex of at least one transition metal selected from transition metals belonging to Group 8 to Group 10 of the Periodic Table;
an optically active cyclic nitrogen-containing compound; and
an acid.

4. The catalyst for homogenous asymmetric hydrogenation according to any one of claims 1 to 3, wherein the optically active cyclic nitrogen-containing compound is a compound represented by the general formula (1): 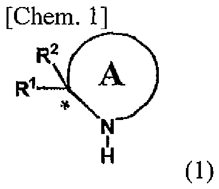 (wherein ring A is a 3- to 7-membered ring which may have a substituent group and comprises at least one kind of an atom selected from the group consisting of carbon, nitrogen, sulfur, oxygen, and phosphorous, and
R¹ and R² each independently represent a hydrogen atom, an alkyl group which may have a substituent group, a cycloalkyl group which may have a substituent group, an alkenyl group which may have a substituent group, an aryl group which may have a substituent group, an aralkyl group which may have a substituent group, an alkoxy group which may have a substituent group, a carboxyl group which may have a substituent group, an alkoxycarbonyl group which may have a substituent group, an amide group which may have a substituent group, a carbamoyl group which may have a substituent group, or a siloxy group which may have a substituent group, wherein R¹ and R² do not represent a same substituent group, and either R¹ or R² may be bonded to the ring A to form a ring; and * represents an asymmetric carbon atom).

5. The catalyst for homogenous asymmetric hydrogenation according to any one of claims 1 to 4, wherein the complex of the transition metal is a complex of a transition metal selected from nickel, ruthenium, rhodium, iridium, palladium and platinum.

6. A manufacturing method of an optically active carbonyl compound, wherein an α, β-unsaturated carbonyl compound is reacted with hydrogen or a hydrogen-donating compound in the presence of the catalyst for homogenous asymmetric hydrogenation according to any one of claims 1 to 5, thereby manufacturing the optically active carbonyl compound.

7. The manufacturing method according to claim 6, wherein the α, β-unsaturated carbonyl compound is an α, β-unsaturated carbonyl compound represented by the general formula (2): 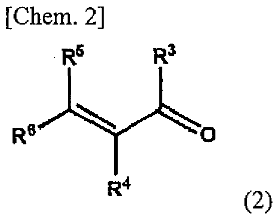 (wherein R³, R⁴, R⁵ and R⁶ each independently represent a hydrogen atom, an alkyl group which may have a substituent group, a cycloalkyl group which may have a substituent group, an alkenyl group which may have a substituent group, an aryl group which may have a substituent group, or an aralkyl group which may have a substituent group, wherein a ring may be formed by R⁵ and R⁶; when R⁴ does not represent a hydrogen atom, R⁵ and R⁶ may be the same; and when R⁴ represents a hydrogen atom, R⁵ and R⁶ do not represent a hydrogen atom and are different from each other); and
the prepared optically active carbonyl compound is an optically active carbonyl compound represented by the general formula (3): 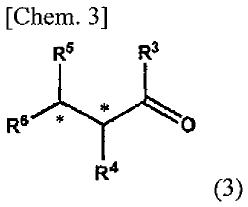 (wherein R³, R⁴, R⁵ and R⁶ are the same as defined in the formula (2), and two * mean that at least one * represents an asymmetric carbon atom).

8. The manufacturing method according to claim 7, wherein the α, β-unsaturated carbonyl compound is geranial, neral or citral.

9. The manufacturing method according to claim 7, wherein the α, β-unsaturated carbonyl compound is cyclic ketones having a 5- to 16-membered ring.

10. A manufacturing method of an optically active carbonyl compound, wherein an α, β-unsaturated carbonyl compound is reacted with hydrogen or a hydrogen-donating compound in the presence of a complex of at least one transition metal selected from transition metals belonging to Group 8 to Group 10 of the Periodic Table and an optically active cyclic nitrogen-containing compound, thereby manufacturing the optically active carbonyl compound.

11. A manufacturing method of an optically active carbonyl compound, wherein an α, β-unsaturated carbonyl compound is reacted with hydrogen or a hydrogen-donating compound in the presence of a complex of at least one transition metal selected from transition metals belonging to Group 8 to Group 10 of the Periodic Table, an optically active cyclic nitrogen-containing compound and an acid, thereby manufacturing the optically active carbonyl compound.

12. The manufacturing method according to claim 10 or 11, wherein the optically active cyclic nitrogen-containing compound is a compound represented by the general formula (1): 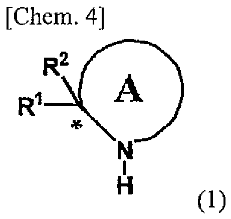 (wherein ring A is a 3- to 7-membered ring which may have a substituent group and comprises at least one kind of an atom selected from the group consisting of carbon, nitrogen, sulfur, oxygen, and phosphorous; and
R¹ and R² each independently represent a hydrogen atom, an alkyl group which may have a substituent group, a cycloalkyl group which may have a substituent group, an alkenyl group which may have a substituent group, an aryl group which may have a substituent group, an aralkyl group which may have a substituent group, an alkoxy group which may have a substituent group, a carboxyl group which may have a substituent group, an alkoxycarbonyl group which may have a substituent group, an amide group which may have a substituent group, a carbamoyl group which may have a substituent group, or a siloxy group which may have a substituent group, wherein R¹ and R² do not represent a same substituent group, and either R¹ or R² may be bonded to the ring A to form a ring; and * represents an asymmetric carbon atom).

13. The manufacturing method according to any one of claims 10 to 12, wherein the optically active cyclic nitrogen-containing compound is a compound represented by the general formula (1): 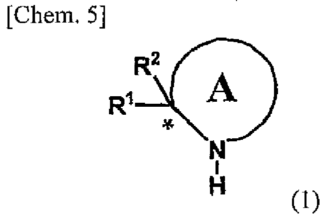 (wherein ring A is a 3- to 7-membered ring which may have a substituent group and comprises at least one atom selected from the group consisting of carbon, nitrogen, sulfur, oxygen, and phosphorous; and
R¹ and R² each independently represent a hydrogen atom, an alkyl group which may have a substituent group, a cycloalkyl group which may have a substituent group, an alkenyl group which may have a substituent group, an aryl group which may have a substituent group, an aralkyl group which may have a substituent group, an alkoxy group which may have a substituent group, a carboxyl group which may have a substituent group, an alkoxycarbonyl group which may have a substituent group, an amide group which may have a substituent group, a carbamoyl group which may have a substituent group, or a siloxy group which may have a substituent group, wherein R¹ and R² do not represent a same substitutent, and either R¹ or R² may be bonded to the ring A to form a ring; and * represents an asymmetric carbon atom).

14. The manufacturing method according to any one of claims 10 to 13, wherein the complex of the transition metal is a complex of a transition metal selected from nickel, ruthenium, rhodium, iridium, palladium and platinum.

15. The manufacturing method according to any one of claims 10 to 14, wherein the α, β-unsaturated carbonyl compound is a compound represented by the general formula (2): 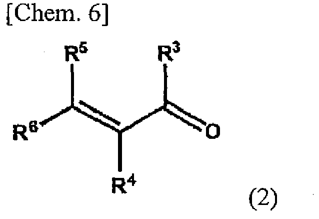 (wherein R³, R⁴, R⁵ and R⁶ each independently represent a hydrogen atom, an alkyl group which may have a substituent group, a cycloalkyl group which may have a substituent group, an alkenyl group which may have a substituent group, an aryl group which may have a substituent group, or an aralkyl group which may have a substituent group, wherein a ring may be formed by R⁵ and R⁶; when R⁴ does not represent a hydrogen atom, R⁵ and R⁶ may be the same; and when R⁴ represents a hydrogen atom, R⁵ and R⁶ do not represent a hydrogen atom and are different from each other), and
the prepared optically active carbonyl compound is an optically active carbonyl compound represented by the general formula (3): 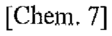 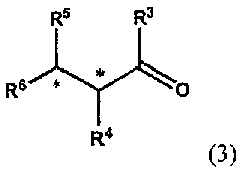 (wherein R³, R⁴, R⁵ and R⁶ are the same as defined in the formula (2), and two * mean that at least one * represents an asymmetric carbon atom).

16. The manufacturing method according to claim 15, wherein the α, β-unsaturated carbonyl compound is geranial, neral or citral.

17. The manufacturing method according to claim 15, wherein the α, β-unsaturated carbonyl compound is cyclic ketones having a 5- to 16-membered ring.
